# EUROPÄISCHE PATENTSCHRIFT

(11) **EP 2 683 686 B1**
(45) Veröffentlichungstag und Bekanntmachung des Hinweises auf die Patenterteilung: **13.05.2015**
(21) Anmeldenummer: 12707089.4
(22) Anmeldetag: 01.03.2012
(51) Int. Cl.: C07C 209/16, C07D 207/20, C07D 207/06, C07C 213/02, C07C 215/08, C07D 265/30, C07D 307/52

(54) **VERFAHREN ZUR HERSTELLUNG VON PRIMÄREN AMINEN DURCH HOMOGEN-KATALYSIERTE ALKOHOLAMINIERUNG**
METHOD FOR THE PREPARATION OF PRIMARY AMINES BY HOMOGENEOUS CATALYSED AMINATION OF ALCOHOLS
METHOD POUR LA PRÉPARATION DES AMINES PRIMAIRES PAR L'AMINATION DES ALCOOLS CATALYSÉE HOMOGÈNE

(30) Priorität: 08.03.2011 EP 11157288
(43) Veröffentlichungstag der Anmeldung: 15.01.2014
(73) Patentinhaber: BASF SE, 67056 Ludwigshafen (DE)
(72) Erfinder: SCHAUB, Thomas, 67433 Neustadt (DE); BUSCHHAUS, Boris, 68161 Mannheim (DE); BRINKS, Marion, Kristina, 68165 Mannheim (DE); SCHELWIES, Mathias, 69115 Heidelberg (DE); PACIELLO, Rocco, 67098 Bad Dürkheim (DE); MELDER, Johann-Peter, 67459 Böhl-Iggelheim (DE); MERGER, Martin, 67227 Frankenthal (DE)
(86) Internationale Anmeldenummer: PCT/EP2012/053582
(87) Internationale Veröffentlichungsnummer: WO 2012/119927

(56) Entgegenhaltungen:
- EP-A2- 0 320 269
- WO-A1-2010/018570

## Beschreibung

Die vorliegende Erfindung betrifft ein Verfahren zur Herstellung von primären Aminen durch Alkoholaminierung von primären Alkoholen mit Ammoniak unter Wasserabspaltung in Gegenwart eines Komplexkatalysators, der mindestens ein Element ausgewählt aus den Gruppen 8 und 9 des Periodensystems sowie mindestens einen Phosphordonorliganden der allgemeinen Formel (I) enthält.

Primäre Amine sind wertvolle Produkte mit einer Vielzahl unterschiedlicher Verwendungen, beispielsweise als Lösungsmittel, Stabilisatoren, zur Synthese von ChelatBildnern, als Edukte zur Herstellung von Kunstharzen, Inhibitoren, grenzflächenaktiven Substanzen, Zwischenprodukte bei der Herstellung von Kraftstoffadditiven (US 3,275,554 A, DE 2125039 A und DE 36 11 230 A), Tensiden, Arznei- und Planzenschutzmitteln, Härtern für Epoxyharze, Katalysatoren für Polyurethane, Zwischenprodukte zur Herstellung quaternärer Ammoniumverbindungen, Weichmachern, Korrosionsinhibitoren, Kunstharzen, Ionenaustauschern, Textilhilfsmitteln, Farbstoffen, Vulkanisationsbeschleunigern und/oder Emulgatoren.

Primäre Amine werden gegenwärtig durch die heterogen-katalysierte Alkoholaminierung von Alkoholen mit Ammoniak hergestellt. Die WO 2008/006752 A1 beschreibt ein Verfahren zur Herstellung von Aminen durch Umsetzung von primären Alkoholen mit Ammoniak in Gegenwart eines heterogenen Katalysators, der Zirkoniumdioxid und Nickel enthält. Die WO 03/051508 A1 betrifft ein Verfahren zur Aminierung von Alkoholen unter Verwendung von spezifischen heterogenen Cu/Ni/Zr/Sn-Katalysatoren. Aus der EP 0 696 572 A1 sind Nickel-, Kupfer-, Zirkonium- und Molybdänoxid enthaltende heterogene Katalysatoren zur Aminierung von Alkoholen mit Ammoniak und Wasserstoff bekannt. Die Reaktionen werden in den vorstehend genannten Dokumenten bei Temperaturen im Bereich von 150 bis 210 °C und Ammoniakdrücken im Bereich von 30 bis 200 bar durchgeführt.

Die homogen-katalysierte Aminierung von Monoalkoholen mit primären und sekundären Aminen ist seit den 1970er Jahren bekannt, wobei meist Ruthenium- oder Iridiumkatalysatoren beschreiben sind. Die homogen-katalysierte Aminierung läuft, im Vergleich zu heterogen-katalysierten Reaktionen, bei deutlich niedrigeren Temperaturen von 100 bis 150°C ab. Die Umsetzung von Alkoholen mit primären und sekundären Aminen ist beispielsweise in folgenden Veröffentlichungen beschrieben: US 3708539; Y. Watanabe, Y. Tsuji, Y. Ohsugi, Tetrahedron Lett. 1981, 22, 2667-2670; S. Bähn, S. Imm, K. Mevius, L. Neubert, A. Tillack, J. M. J. Williams, M. Beller, Chem. Eur. J. 2010**,** DOI: 10.1002/chem.200903144; A. Tillack, D. Hollmann, D. Michalik, M. Beller, Tetrahedron Lett. 2006, 47, 8881-8885; D. Hollmann, S. Bähn, A. Tillack, M. Beller, Angew. Chem. Int. Ed. 2007, 46, 8291-8294; A. Tillack, D. Hollmann, K. Mevius, D. Michalik, S. Bähn, M. Beller, Eur. J. Org. Chem. 2008, 4745-4750; M. H. S. A. Hamid, C. L. Allen, G. W. Lamb, A. C. Maxwell, H. C. Maytum, A. J. A. Watson, J. M. J. Williams, J. Am. Chem. Soc. 2009, 131, 1766-1774; O. Saidi, A. J. Blacker, M. M. Farah, S. P. Marsden, J. M. J. Williams, Chem. Commun. 2010, 46, 1541-1543; A. Tillack, D. Hollmann, D. Michalik, M. Beller, Tet. Lett. 2006, 47, 8881-8885; A. Del Zlotto, W. Baratta, M. Sandri, G. Verardo, P. Rigo, Eur. J. Org. Chem. 2004, 524-529; A. Fujita, Z. Li, N. Ozeki, R. Yamaguchi, Tetrahedron Lett. 2003, 44, 2687-2690; Y. Watanabe, Y. Morisaki, T. Kondo, T. Mitsudo J. Org. Chem. 1996, 61, 4214-4218, B. Blank, M. Madalska, R. Kempe, Adv. Synth. Catal. 2008, 350, 749-750, A. Martinez-Asencio, D. J. Ramon, M. Yus, Tetrahedron Lett. 2010, 51, 325-327. Der größte Nachteil der vorstehend beschriebenen Systeme ist, dass mit diesen Verfahren nur die Aminierung von Alkoholen mit primären und sekundären Aminen unter Bildung von sekundären und tertiären Aminen möglich ist. Die Umsetzung von Alkoholen mit Ammoniak, welches die wirtschaftlich attraktivste Aminierungsreaktion darstellt, wird in diesen Arbeiten nicht beschrieben.

S. Imm, S. Bähn, L. Neubert, H. Neumann, M. Beller, Angew. Chem. 2010, 122, 8303-8306 und D. Pingen, C. Müller, D. Vogt, Angew. Chem. 2010, 122, 8307-8310 beschreiben die mit Rutheniumkatalysatoren homogen-katalysierte Aminierung von sekundären Alkoholen wie Cyclohexanol mit Ammoniak. EP 0 320 269 A2 offenbart die Palladium katalysierte Aminierung von primären Allylalkohlen mit Ammoniak unter Erhalt primärer Allylamine. WO 2010/018570 und C. Gunanathan, D. Milstein, Angew. Chem. Int. Ed. 2008, 47, 8661-8664 beschreiben die Aminierung von primären Alkoholen mit Ammoniak zu primären Aminen mit Hilfe eines speziellen Rutheniumkatalysators mit Acridin-basierten Pinzettenliganden.

R. Kawahara, K.I. Fujita, R. Yamaguchi, J. Am. Chem. Soc. DOI: 10.1021/ja107274w beschreibt die Aminierung primärer Alkohole mit Ammoniak unter Verwendung eines Iridiumkatalysators, der als Liganden Cp* (1,2,3,4,5-Pentamethylcyclopentadienyl) und Ammoniak aufweist. Mit dem dort beschriebenen Katalysatorsystem werden beim Umsatz primärer Alkohole mit Ammoniak jedoch ausschließlich die unerwünschten tertiären Amine erhalten.

EP 0 234 401 A1 beschreibt die Umsetzung von Diethylenglykol mit Ammoniak in Gegenwart einer Rutheniumcarbonylverbindung. Bei dem in EP 0 234 401 A1 beschriebenen Verfahren bildet sich das Monoaminierungsprodukt (Monoethanolamin). Nachteilig ist jedoch, dass sich als Nebenprodukte die sekundären und tertiären Amine (Di-und Triethanolamin) und zyklische Produkte (N-(Hydroxyethyl)Piperazin und N,N'-Bis(hydroxyethyl)piperazin) bilden.

Obwohl im Stand der Technik Verfahren zur homogen-katalysierten Umsetzung von primären Alkoholen zu primären Aminen beschrieben sind, besteht dennoch ein großer Bedarf an alternativen, verbesserten Herstellungsverfahren mit einfacheren und leicht zugänglichen Phosphanliganden.

Aufgabe der vorliegenden Erfindung ist es, ein Verfahren zur Herstellung von primären Aminen durch homogen-katalysierte Alkoholaminierung von primären Alkoholen mit Ammoniak unter Wasserabspaltung bereitzustellen, bei dem kein Komplexkatalysator auf Acridin-Basis eingesetzt wird und bei dem die Bildung unerwünschter Nebenprodukte wie sekundärer und tertiärer Amine sowie zyklischer Amine weitestgehend vermieden wird.

Gelöst wird die Aufgabe durch ein Verfahren zur Herstellung von primären Aminen, die mindestens eine funktionelle Gruppe der Formel (-CH₂-NH₂) aufweisen, durch Alkoholaminierung von Edukten, die mindestens eine funktionelle Gruppe der Formel (-CH₂-OH) aufweisen, mit Ammoniak unter Wasserabspaltung, wobei die Alkoholaminierung homogen-katalysiert in Gegenwart mindestens eines Komplexkatalysators, der mindestens ein Element ausgewählt aus den Gruppen 8 und 9 des Periodensystems sowie mindestens einen Phosphordonorliganden der allgemeinen Formel (I) enthält, 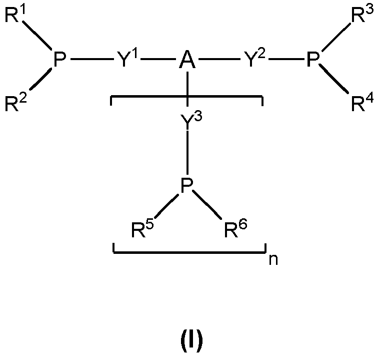 durchgeführt wird, wobei
- n: ist 0 oder 1;
- R¹, R², R³, R⁴, R⁵, R⁶: sind unabhängig voneinander unsubstituiertes oder zumindest monosubstituiertes C₁-C₁₀-Alkyl, C₁-C₄-Alkyldiphenylphosphin (-C₁-C₄-Alkyl-P(Phenyl)₂), C₃-C₁₀-Cycloalkyl, C₃-C₁₀-Heterocyclyl enthaltend mindestens ein Heteroatom ausgewählt aus N, O und S, C₅-C₁₄-Aryl oder C₅-C₁₀-Heteroaryl enthaltend mindestens ein Heteroatom ausgewählt aus N, O und S,
wobei die Substituenten ausgewählt sind aus der Gruppe bestehend aus: F, Cl, Br, OH, CN, NH₂ und C₁-C₁₀-Alkyl;
- A: ist
i) eine Brückengruppe ausgewählt aus der Gruppe unsubstituiertes oder zumindest monosubstituiertes N, O, P, C₁-C₆-Alkan, C₃-C₁₀-Cycloalkan, C₃-C₁₀-Heterocycloalkan enthaltend mindestens ein Heteroatom ausgewählt aus N, O und S, C₅-C₁₄-Aromat und C₅-C₆-Heteroaromat enthaltend mindestens ein Heteroatom ausgewählt aus N, O und S.
   wobei die Substituenten ausgewählt sind aus der Gruppe bestehend aus: C₁-C₄-Alkyl, Phenyl, F, Cl, Br, OH, OR⁷, NH₂, NHR⁷ oder N(R⁷)₂,
   wobei R⁷ ausgewählt ist aus C₁-C₁₀-Alkyl und C₅-C₁₀-Aryl;
   oder
ii) eine Brückengruppe der Formel II oder III: 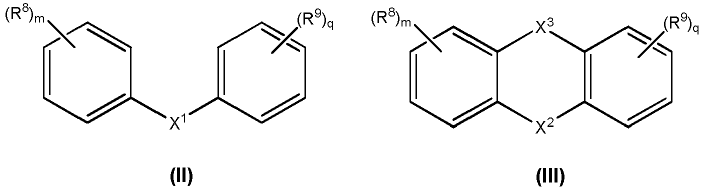
   m, q sind unabhängig voneinander 0, 1, 2, 3 oder 4;
   R⁸, R⁹ sind unabhängig voneinander ausgewählt aus der Gruppe C₁-C₁₀-Alkyl, F, Cl, Br, OH, OR⁷, NH₂, NHR⁷ und N(R⁷)₂,
   wobei R⁷ ausgewählt ist aus C₁-C₁₀-Alkyl und C₅-C₁₀-Aryl;
   X¹, X² sind unabhängig voneinander NH, O oder S;
   X³ ist eine Bindung, NH, NR¹⁰, O, S oder CR¹¹R¹²;
   R¹⁰ ist unsubstituiertes oder zumindest monosubstituiertes C₁-C₁₀-Alkyl, C₃-C₁₀-Cycloalkyl, C₃-C₁₀-Heterocyclyl enthaltend mindestens ein Heteroatom ausgewählt aus N, O und S, C₅-C₁₄-Aryl oder C₅-C₁₀-Heteroaryl enthaltend mindestens ein Heteroatom ausgewählt aus N, O und S,
   wobei die Substituenten ausgewählt sind aus der Gruppe bestehend aus: F, Cl, Br, OH, CN, NH₂ und C₁-C₁₀-Alkyl;
   R¹¹, R¹² sind unabhängig voneinander unsubstituiertes oder zumindest monosubstituiertes C₁-C₁₀-Alkyl, C₁-C₁₀-Alkoxy, C₃-C₁₀-Cycloalkyl, C₃-C₁₀-Cycloalkoxy, C₃-C₁₀-Heterocyclyl enthaltend mindestens ein Heteroatom ausgewählt aus N, O und S, C₅-C₁₄-Aryl, C₅-C₁₄-Aryloxy oder C₅-C₁₀-Heteroaryl enthaltend mindestens ein Heteroatom ausgewählt aus N, O und S,
   wobei die Substituenten ausgewählt sind aus der Gruppe bestehend aus: F, Cl, Br, OH, CN, NH₂ und C₁-C₁₀-Alkyl;
- Y¹, Y², Y³: sind unabhängig voneinander eine Bindung, unsubstituiertes oder zumindest monosubstituiertes Methylen, Ethylen, Trimethylen, Tetramethylen, Pentamethylen oder Hexamethylen,
wobei die Substituenten ausgewählt sind aus der Gruppe bestehend aus: F, Cl, Br, OH, OR⁷, CN, NH₂, NHR⁷, N(R⁷)₂ und C₁-C₁₀-Alkyl,
wobei R⁷ ausgewählt ist aus C₁-C₁₀-Alkyl und C₅-C₁₀-Aryl.

Überraschend wurde festgestellt, dass mit den im erfindungsgemäßen Verfahren eingesetzten Komplexkatalysatoren, die mindestens ein Element ausgewählt aus den Gruppen 8 und 9 des Periodensystems sowie mindestens einen Phosphordonorliganden der allgemeinen Formel (I) enthalten, primäre Amine gegenüber den im Stand der Technik beschriebenen Verfahren, in deutlich verbesserten Ausbeuten erhalten werden. Darüber hinaus wird die Bildung unerwünschter Nebenprodukte wie sekundärer und tertiärer Amine sowie zyklischer Amine weitestgehend vermieden.

### Edukte

Im erfindungsgemäßen Verfahren werden als Edukte Alkohole eingesetzt, die mindestens eine funktionelle Gruppe der Formel (-CH₂-OH) aufweisen.

Als Edukte sind praktisch alle Alkohole geeignet, die die vorstehend genannten Voraussetzungen erfüllen. Die Alkohole können geradkettig, verzweigt oder zyklisch sein. Die Alkohole können darüber hinaus Substituenten tragen, die sich unter den Reaktionsbedingungen der Alkoholaminierung inert verhalten, beispielsweise Alkoxy, Alkenyloxy, Alkylamino, Dialkylamino und Halogene (F, Cl, Br, I). Als Edukte können erfindungsgemäß neben Monoalkoholen auch Diole, Triole, Polyole und Alkanolamine eingesetzt werden, die mindestens eine funktionelle Gruppe der Formel (-CH₂-OH) aufweisen.

Erfindungsgemäß einzusetzende Monoalkohole sind Alkohole, die nur eine funktionelle Gruppe der Formel (-CH₂-OH) aufweisen. Erfindungsgemäß einzusetzende Diole, Triole und Polyole sind Alkohole, die mindestens eine funktionelle Gruppe der Formel (-CH₂-OH) und eine, zwei oder mehrere weitere Hydroxylgruppen aufweisen. Erfindungsgemäß einzusetzende Alkanolamine sind Verbindungen, die mindestens eine funktionelle Gruppe der Formel (-CH₂-OH) und mindestens eine weitere primäre, sekundäre, oder tertiäre Aminogruppe aufweisen.

Geeignete Alkohole sind beispielsweise solche der allgemeinen Formel (IV):

R^{a}-CH₂-OH (IV),

wobei
- R^{a}: ist ausgewählt aus der Gruppe Wasserstoff, unsubstituiertes oder zumindest monosubstituiertes C₁-C₃₀-Alkyl, C₃-C₁₀-Cycloalkyl, C₃-C₁₀-Heterocyclyl enthaltend mindestens ein Heteroatom ausgewählt aus N, O und S, C₅-C₁₄-Aryl und C₅-C₁₄-Heteroaryl enthaltend mindestens ein Heteroatom ausgewählt aus N, O und S,
wobei die Substituenten ausgewählt sind aus der Gruppe bestehend aus: F, Cl, Br, OH, OR⁷, CN, NH₂, NHR⁷ oder N(R⁷)₂, C₁-C₁₀-Alkyl, C₃-C₁₀-Cycloalkyl, C₃-C₁₀-Heterocyclyl enthaltend mindestens ein Heteroatom ausgewählt aus N, O und S, C₅-C₁₄-Aryl und C₅-C₁₄-Heteroaryl enthaltend mindestens ein Heteroatom ausgewählt aus N, O und S.
wobei R⁷ ausgewählt ist aus C₁-C₁₀-Alkyl und C₅-C₁₀-Aryl.

Bevorzugt werden beispielsweise die folgenden Alkohole aminiert: Methanol, Ethanol, n-Propanol, n-Butanol, iso-Butanol, n-Pentanol, n-Hexanol, n-Heptanol, n-Octanol, n-Nonanol, 2-Ethylhexanol, Tridecanol, Stearylalkohol, Palmitylalkohol, Benzylalkohol, 2-Phenyl-ethanol, 2-(p-Methoxyphenyl)ethanol, 2-(3,4-Dimethoxyphenyl)ethanol, Allylalkohol, Propargylalkohol, 2-Hydroxymethylfuran, Hydroxymethylfurfural, Milchsäure und Serin.

Als Edukte können alle bekannten Diole eingesetzt werden, die mindestens eine funktionelle Gruppe der Formel (-CH₂-OH) aufweisen. Beispiele für Diole, die als Edukte in das erfindungsgemäße Verfahren eingesetzt werden können, sind 1,2-Ethandiol (Ethylenglykol), 1,2-Propandiol (1,2-Propylenglykol), 1,3-Propandiol (1,3-Propylenglykol), 1,4-Butandiol (1,4-Butylenglykol), 1,2-Butandiol (1,2-Butylenglykol), 2,3-Butandiol, 2-Methyl-1,3-propandiol, 2,2-Dimethyl-1,3-propandiol (Neopentylglykol), 1,5-Pentandiol, 1,2-Pentandiol, 1,6-Hexandiol, 1,2-Hexandiol, 1,7-Heptandiol, 1,2-Heptandiol, 1,8-Oktandiol, 1,2-Oktandiol, 1,9-Nonandiol, 1,2-Nonandiol, 1,10-Decandiol, 2,4-Dimethyl-2,5-hexandiol, Hydroxypivalinsäureneopentylglykolester, Diethylenglykol, Triethylenglykol, 2-Buten-1,4-diol, 2-Butin-1,4-diol, Polyethylenglykole, Polypropylenglykole wie 1,2-Polypropylenglykol und 1,3-Polypropylenglykol, Polytetrahydrofuran, Diethanolamin, 1,4-Bis-(2-hydroxyethyl)piperazin, Diisopropanolamin, N-Butyldiethanolamin, 2,5-(Dimethanol)furan, 1,4-Bis(hydroxymethyl)cyclohexan und N-Methyldiethanolamin. 2,5-(Dimethanol)furan wird auch als 2,5-Bis(hydroxymethyl)furan bezeichnet.

Bevorzugt sind Diole, die zwei funktionelle Gruppen der Formel (-CH₂-OH) aufweisen.

Insbesondere bevorzugte Diole sind 1,2-Ethandiol (Ethylenglykol), 1,2-Propandiol (1,2-Propylenglykol), 1,3-Propandiol (1,3-Propylenglykol), 1,4-Butandiol (1,4-Butylenglykol), 2-Methyl-1,3-propandiol, 2,2-Dimethyl-1,3-propandiol (Neopentylglykol), 1,5-Pentandiol, 1,6-Hexandiol, 1,7-Heptandiol, 1,8-Oktandiol, 1,9-Nonandiol, Diethylenglykol, Triethylenglykol, Poylyethylenglykole, Polypropylenglykole wie 1,2-Polypropylenglykol und 1,3 Polypropylenglykol, Polytetrahydrofuran, Diethanolamin, Diisopropanolamin, N-Butyldiethanolamin, 2,5-(Dimethanol)furan und N-Methyldiethanolamin.

Als Edukte können alle bekannten Triole eingesetzt werden, die mindestens eine funktionelle Gruppe der Formel (-CH₂-OH) aufweisen. Beispiele für Triole, die als Edukte in das erfindungsgemäße Verfahren eingesetzt werden können, sind Glycerin, Trimethylolpropan und Triethanolamin.

Bevorzugt sind Triole, die mindestens zwei funktionelle Gruppen der Formel (-CH₂-OH) aufweisen.

Insbesondere bevorzugte Triole sind Glycerin und Triethanolamin.

Als Edukte können alle bekannten Polyole eingesetzt werden, die mindestens eine funktionelle Gruppe der Formel (-CH₂-OH) aufweisen. Beispiele für Polyole, die als Edukte in das erfindungsgemäße Verfahren eingesetzt werden können, sind 2,2-Bis(hydroxymethyl)-1,3-propandiol (Pentaerythrit), Sorbit, Inositol, Zucker und Polymere mit primären Hydroxylgruppen (-CH₂-OH) wie beispielsweise Glucose, Mannose, Fructose, Ribose, Desoxyribose, Galactose, N-Acetyl-Glucosamin, Fucose, Rhamnose, Saccharose, Lactose, Cellobiose, Maltose und Amylose, Cellulose, Stärke und Xanthan.

Bevorzugt sind Polyole, die mindestens zwei funktionelle Gruppen der Formel (-CH₂-OH) aufweisen.

Insbesondere bevorzugte Polyole sind Cellulose, Polyvinylalkohol und Glucose.

Als Edukte können alle bekannten Alkanolamine eingesetzt werden, die mindestens eine primäre Hydroxylgruppe (-CH₂-OH) aufweisen. Beispiele für Alkanolamine, die als Edukte in das erfindungsgemäße Verfahren eingesetzt werden können, sind Monoaminoethanol, 3-Aminopropan-1-ol, 2-Aminopropan-1-ol, 4-Aminobutan-1-ol, 2-Aminobutan-1-ol, 3-Aminobutan-1-ol, 5-Aminopentan-1-ol, 2-Aminopentan-1-ol, 6-Aminohexan-1-ol, 2-Aminohexan-1-ol, 7-Aminoheptan-1-ol, 2-Aminoheptan-1-ol, 8-Aminooctan-1-ol, 2-Aminooctan-1-ol, N-(2-Hydroxyethyl)anilin, N-(2-Aminoethyl)ethanolamin, 1-(2-Hydroxyethyl)piperazin, 2-(2-Aminoethoxy)ethanol, N-Butylethanolamin, N-Ethylethanolamin, N-Methylethanolamin N,N-Dimethylethanolamin, N-(2-Hydroxyethyl)-1,3-propandiamin, 3-(2-Hydroxyethyl)amino-1-propanol, 3-Dimethylamino-1-propanol, N,N-Dibutylethanolamin, N,N-Dimethyl-isopropylamin und N,N-Diethylethanolamin.

Bevorzugt sind Alkanolamine, die mindestens eine primäre Hydroxylgruppe (-CH₂-OH) und mindestens eine primäre Aminogruppe der Formel (-CH₂-NH₂) aufweisen.

Insbesondere bevorzugtes Alkanolamin ist Monoaminoethanol.

### Komplexkatalysator

Im erfindungsgemäßen Verfahren wird mindestens ein Komplexkatalysator, der mindestens ein Element ausgewählt aus den Gruppen 8 und 9 des Periodensystems (Nomenklatur gemäß IUPAC) sowie mindestens einen Phosphordonorliganden der allgemeinen Formel (I) enthält, 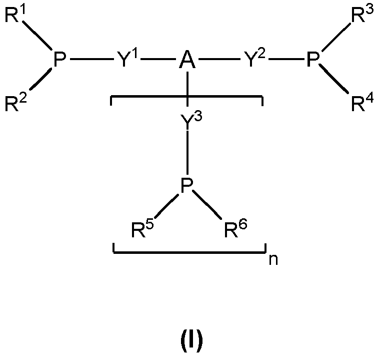 eingesetzt, wobei
- n: ist 0 oder 1;
- R¹, R², R³, R⁴, R⁵, R⁶: sind unabhängig voneinander unsubstituiertes oder zumindest monosubstituiertes C₁-C₁₀-Alkyl, C₁-C₄-Alkyldiphenylphosphin (-C₁-C₄-Alkyl-P(Phenyl)₂), C₃-C₁₀-Cycloalkyl, C₃-C₁₀-Heterocyclyl enthaltend mindestens ein Heteroatom ausgewählt aus N, O und S, C₅-C₁₄-Aryl oder C₅-C₁₀-Heteroaryl enthaltend mindestens ein Heteroatom ausgewählt aus N, O und S,
wobei die Substituenten ausgewählt sind aus der Gruppe bestehend aus: F, Cl, Br, OH, CN, NH₂ und C₁-C₁₀-Alkyl;
- A: ist
i) eine Brückengruppe ausgewählt aus der Gruppe unsubstituiertes oder zumindest monosubstituiertes N, O, P, C₁-C₆-Alkan, C₃-C₁₀-Cycloalkan, C₃-C₁₀-Heterocycloalkan enthaltend mindestens ein Heteroatom ausgewählt aus N, O und S, C₅-C₁₄-Aromat und C₅-C₆-Heteroaromat enthaltend mindestens ein Heteroatom ausgewählt aus N, O und S,
   wobei die Substituenten ausgewählt sind aus der Gruppe bestehend aus: C₁-C₄-Alkyl, Phenyl, F, Cl, Br, OH, OR⁷, NH₂, NHR⁷ oder N(R⁷)₂,
   wobei R⁷ ausgewählt ist aus C₁-C₁₀-Alkyl und C₅-C₁₀-Aryl;
   oder
ii) eine Brückengruppe der Formel II oder III: 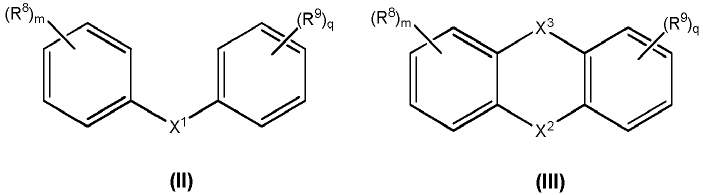
   m, q sind unabhängig voneinander 0, 1, 2, 3 oder 4;
   R⁸, R⁹ sind unabhängig voneinander ausgewählt aus der Gruppe C₁-C₁₀-Alkyl, F, Cl, Br, OH, OR⁷, NH₂, NHR⁷ und N(R⁷)₂,
   wobei R⁷ ausgewählt ist aus C₁-C₁₀-Alkyl und C₅-C₁₀-Aryl;
   X¹, X² sind unabhängig voneinander NH, O oder S;
   X³ ist eine Bindung, NH, NR¹⁰, O, S oder CR¹¹R¹²;
   R¹⁰ ist unsubstituiertes oder zumindest monosubstituiertes C₁-C₁₀-Alkyl, C₃-C₁₀-Cycloalkyl, C₃-C₁₀-Heterocyclyl enthaltend mindestens ein Heteroatom ausgewählt aus N, O und S, C₅-C₁₄-Aryl oder C₅-C₁₀-Heteroaryl enthaltend mindestens ein Heteroatom ausgewählt aus N, O und S,
   wobei die Substituenten ausgewählt sind aus der Gruppe bestehend aus: F, Cl, Br, OH, CN, NH₂ und C₁-C₁₀-Alkyl;
   R¹¹, R¹² sind unabhängig voneinander unsubstituiertes oder zumindest monosubstituiertes C₁-C₁₀-Alkyl, C₁-C₁₀-Alkoxy, C₃-C₁₀-Cycloalkyl, C₃-C₁₀-Cycloalkoxy,C₃-C₁₀-Heterocyclyl enthaltend mindestens ein Heteroatom ausgewählt aus N, O und S, C₅-C₁₄-Aryl, C₅-C₁₄-Aryloxy oder C₅-C₁₀-Heteroaryl enthaltend mindestens ein Heteroatom ausgewählt aus N, O und S,
   wobei die Substituenten ausgewählt sind aus der Gruppe bestehend aus: F, Cl, Br, OH, CN, NH₂ und C₁-C₁₀-Alkyl;
- Y¹, Y², Y³: sind unabhängig voneinander eine Bindung, unsubstituiertes oder zumindest monosubstituiertes Methylen, Ethylen, Trimethylen, Tetramethylen, Pentamethylen oder Hexamethylen,
wobei die Substituenten ausgewählt sind aus der Gruppe bestehend aus: F, Cl, Br, OH, OR⁷, CN, NH₂, NHR⁷, N(R⁷)₂ und C₁-C₁₀-Alkyl,
wobei R⁷ ausgewählt ist aus C₁-C₁₀-Alkyl und C₅-C₁₀-Aryl.

A ist erfindungsgemäß eine Brückengruppe. Für den Fall, dass A ausgewählt ist aus der Gruppe unsubstituiertes oder zumindest monosubstituiertes C₁-C₆-Alkan, C₃-C₁₀-Cycloalkan, C₃-C₁₀ Heterocycloalkan, C₅-C₁₄-Aromat und C₅-C₆-Heteroaromat und Brückengruppen der Formel (II) oder (III), sind für den Fall (n = 0) zwei Wasserstoffatome der Brückengruppe durch Bindungen zu den benachbarten Substituenten Y¹ und Y² ersetzt. Für den Fall (n = 1) sind drei Wasserstoffatome der Brückengruppe durch drei Bindungen zu den benachbarten Substituenten Y¹, Y² und Y³ ersetzt.

Für den Fall, dass A P (Phosphor) ist, bildet der Phosphor für den Fall (n=0) zwei Bindungen zu den benachbarten Substituenten Y¹ und Y² und eine Bindung zu einem Substituenten ausgewählt aus der Gruppe bestehend aus C₁-C₄-Alkyl und Phenyl aus. Für den Fall (n=1) bildet der Phosphor drei Bindungen zu den benachbarten Substituenten Y¹, Y² und Y³ aus.

Für den Fall, dass A N (Stickstoff) ist, bildet der Stickstoff für den Fall (n = 0) zwei Bindungen zu den benachbarten Substituenten Y¹ und Y² und eine Bindung zu einem Substituenten ausgewählt aus der Gruppe bestehend aus C₁-C₄-Alkyl und Phenyl aus. Für den Fall (n = 1) bildet der Stickstoff drei Bindungen zu den benachbarten Substituenten Y¹, Y² und Y³ aus.

Für den Fall, dass A O (Sauerstoff) ist, ist n = 0. Der Sauerstoff bildet zwei Bindungen zu den benachbarten Substituenten Y¹ und Y² aus.

Die Elemente der Gruppen 8 und 9 des Periodensystems umfassen Eisen, Cobalt, Ruthenium, Rhodium, Osmium und Iridium. Bevorzugt sind Komplexkatalysatoren, die mindestens ein Element ausgewählt aus Ruthenium und Iridium enthalten.

In einer bevorzugten Ausführungsform wird das erfindungsgemäße Verfahren in Gegenwart mindestens eines Komplexkatalysators, der mindestens ein Element ausgewählt aus den Gruppen 8 und 9 des Periodensystems sowie mindestens einen Phosphordonorliganden der allgemeinen Formel (I) enthält, durchgeführt, wobei
- n: ist 0 oder 1;
- R¹, R², R³, R⁴, R⁵, R⁶: sind unabhängig voneinander unsubstituiertes C₁-C₁₀-Alkyl, C₃-C₁₀-Cycloalkyl, C₃-C₁₀-Heterocyclyl enthaltend mindestens ein Heteroatom ausgewählt aus N, O und S, C₅-C₁₄-Aryl oder C₅-C₁₀-Heteroaryl enthaltend mindestens ein Heteroatom ausgewählt aus N, O und S;
- A: ist
i) eine Brückengruppe ausgewählt aus der Gruppe unsubstituierte(r)s C₁-C₆-Alkan, C₃-C₁₀-Cycloalkan, C₃-C₁₀-Heterocycloalkan enthaltend mindestens ein Heteroatom ausgewählt aus N, O und S, C₅-C₁₄-Aromat und C₅-C₆-Heteroaromat enthaltend mindestens ein Heteroatom ausgewählt aus N, O und S;
   oder
ii) eine Brückengruppe der Formel (II) oder (III): 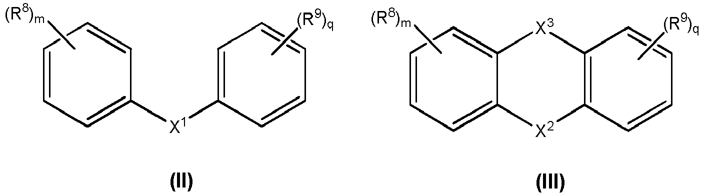
   m, q sind unabhängig voneinander 0, 1, 2, 3 oder 4;
   R⁸, R⁹ sind unabhängig voneinander ausgewählt aus der Gruppe C₁-C₁₀-Alkyl, F, Cl, Br, OH, OR⁷, NH₂, NHR⁷ und N(R⁷)₂,
   wobei R⁷ ausgewählt ist aus C₁-C₁₀-Alkyl und C₅-C₁₀-Aryl;
   X¹, X² sind unabhängig voneinander NH, O oder S;
   X³ ist eine Bindung, NH, NR¹⁰, O, S oder CR¹¹R¹²;
   R¹⁰ ist unsubstituiertes C₁-C₁₀-Alkyl, C₃-C₁₀-Cycloalkyl, C₃-C₁₀-Heterocyclyl enthaltend mindestens ein Heteroatom ausgewählt aus N, O und S, C₅-C₁₄-Aryl oder C₅-C₁₀-Heteroaryl enthaltend mindestens ein Heteroatom ausgewählt aus N, O und S;
   R¹¹, R¹² sind unabhängig voneinander unsubstituiertes C₁-C₁₀-Alkyl, C₁-C₁₀-Alkoxy, C₃-C₁₀-Cycloalkyl, C₃-C₁₀-Cycloalkoxy,C₃-C₁₀-Heterocyclyl enthaltend mindestens ein Heteroatom ausgewählt aus N, O und S, C₅-C₁₄-Aryl, C₅-C₁₄-Aryloxy oder C₅-C₁₀-Heteroaryl enthaltend mindestens ein Heteroatom ausgewählt aus N, O und S;
- Y¹, Y², Y³: sind unabhängig voneinander eine Bindung, unsubstituiertes Methylen, Ethylen, Trimethylen, Tetramethylen, Pentamethylen oder Hexamethylen.

In einer weiteren bevorzugten Ausführungsform wird das erfindungsgemäße Verfahren in Gegenwart mindestens eines Komplexkatalysators, der mindestens ein Element ausgewählt aus den Gruppen 8 und 9 des Periodensystems sowie mindestens einen Phosphordonorliganden der allgemeinen Formel (V) enthält, 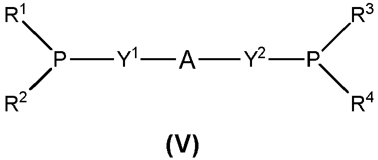 durchgeführt, wobei
- R¹, R², R³, R⁴: sind unabhängig voneinander unsubstituiertes oder zumindest monosubstituiertes C₁-C₁₀-Alkyl, C₁-C₄-Alkyldiphenylphosphin (-C₁-C₄-Alkyl-P(Phenyl)₂), C₃-C₁₀-Cycloalkyl, C₃-C₁₀-Heterocyclyl enthaltend mindestens ein Heteroatom ausgewählt aus N, O und S, C₅-C₁₄-Aryl oder C₅-C₁₀-Heteroaryl enthaltend mindestens ein Heteroatom ausgewählt aus N, O und S,
wobei die Substituenten ausgewählt sind aus der Gruppe bestehend aus: F, Cl, Br, OH, CN, NH₂ und C₁-C₁₀-Alkyl;
- A: ist
i) eine Brückengruppe ausgewählt aus der Gruppe unsubstituiertes oder zumindest monosubstituiertes N, O, P, C₁-C₆-Alkan, C₃-C₁₀-Cycloalkan, C₃-C₁₀-Heterocycloalkan enthaltend mindestens ein Heteroatom ausgewählt aus N, O und S, C₅-C₁₄-Aromat und C₅-C₆-Heteroaromat enthaltend mindestens ein Heteroatom ausgewählt aus N, O und S,
   wobei die Substituenten ausgewählt sind aus der Gruppe bestehend aus: C₁-C₄-Alkyl, Phenyl, F, Cl, Br, OH, OR⁷, NH₂, NHR⁷ oder N(R⁷)₂,
   wobei R⁷ ausgewählt ist aus C₁-C₁₀-Alkyl und C₅-C₁₀-Aryl;
   oder
ii) eine Brückengruppe der Formel II oder III: 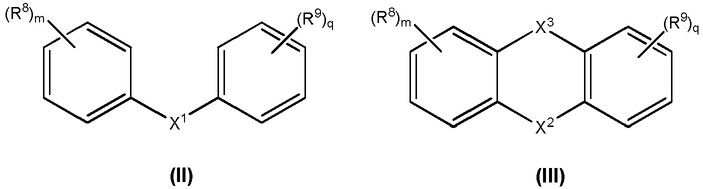
   m, q sind unabhängig voneinander 0, 1, 2, 3 oder 4;
   R⁸, R⁹ sind unabhängig voneinander ausgewählt aus der Gruppe C₁-C₁₀-Alkyl, F, Cl, Br, OH, OR⁷, NH₂, NHR⁷ und N(R⁷)₂,
   wobei R⁷ ausgewählt ist aus C₁-C₁₀-Alkyl und C₅-C₁₀-Aryl;
   X¹, X² sind unabhängig voneinander NH, O oder S,
   X³ ist eine Bindung, NH, NR¹⁰, O, S oder CR¹¹R¹²;
   R¹⁰ ist unsubstituiertes oder zumindest monosubstituiertes C₁-C₁₀-Alkyl, C₃-C₁₀-Cycloalkyl, C₃-C₁₀-Heterocyclyl enthaltend mindestens ein Heteroatom ausgewählt aus N, O und S, C₅-C₁₄-Aryl oder C₅-C₁₀-Heteroaryl enthaltend mindestens ein Heteroatom ausgewählt aus N, O und S,
   wobei die Substituenten ausgewählt sind aus der Gruppe bestehend aus: F, Cl, Br, OH, CN, NH₂ und C₁-C₁₀-Alkyl;
   R¹¹, R¹² sind unabhängig voneinander unsubstituiertes oder zumindest monosubstituiertes C₁-C₁₀-Alkyl, C₁-C₁₀-Alkoxy, C₃-C₁₀-Cycloalkyl, C₃-C₁₀-Cycloalkoxy,C₃-C₁₀-Heterocyclyl enthaltend mindestens ein Heteroatom ausgewählt aus N, O und S, C₅-C₁₄-Aryl, C₅-C₁₄-Aryloxy oder C₅-C₁₀-Heteroaryl enthaltend mindestens ein Heteroatom ausgewählt aus N, O und S,
   wobei die Substituenten ausgewählt sind aus der Gruppe bestehend aus: F, Cl, Br, OH, CN, NH₂ und C₁-C₁₀-Alkyl;
- Y¹, Y²: sind unabhängig voneinander eine Bindung, unsubstituiertes oder zumindest monosubstituiertes Methylen, Ethylen, Trimethylen, Tetramethylen, Pentamethylen oder Hexamethylen,
wobei die Substituenten ausgewählt sind aus der Gruppe bestehend aus: F, Cl, Br, OH, OR⁷, CN, NH₂, NHR⁷, N(R⁷)₂ und C₁-C₁₀-Alkyl,
wobei R⁷ ausgewählt ist aus C₁-C₁₀-Alkyl und C₅-C₁₀-Aryl.

In einer weiteren bevorzugten Ausführungsform wird das erfindungsgemäße Verfahren in Gegenwart mindestens eines Komplexkatalysators, der mindestens ein Element ausgewählt aus den Gruppen 8 und 9 des Periodensystems sowie mindestens einen Phosphordonorliganden der allgemeinen Formel (VI) enthält, 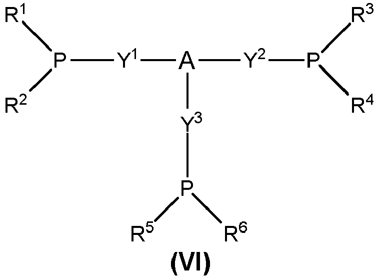 durchgeführt, wobei
- R¹, R², R³, R⁴, R⁵, R⁶: sind unabhängig voneinander unsubstituiertes oder zumindest monosubstituiertes C₁-C₁₀-Alkyl, C₁-C₄-Alkyldiphenylphosphin, C₃-C₁₀-Cycloalkyl, C₃-C₁₀-Heterocyclyl enthaltend mindestens ein Heteroatom ausgewählt aus N, O und S, C₅-C₁₄-Aryl oder C₅-C₁₀-Heteroaryl enthaltend mindestens ein Heteroatom ausgewählt aus N, O und S,
wobei die Substituenten ausgewählt sind aus der Gruppe bestehend aus: F, Cl, Br, OH, CN, NH₂ und C₁-C₁₀-Alkyl;
- A: ist eine Brückengruppe ausgewählt aus der Gruppe unsubstituiertes oder zumindest monosubstituiertes N, P, C₁-C₆-Alkan, C₃-C₁₀-Cycloalkan, C₃-C₁₀-Heterocycloalkan enthaltend mindestens ein Heteroatom ausgewählt aus N, O und S, C₅-C₁₄-Aromat und C₅-C₆-Heteroaromat enthaltend mindestens ein Heteroatom ausgewählt aus N, O und S,
wobei die Substituenten ausgewählt sind aus der Gruppe bestehend aus: C₁-C₄-Alkyl, Phenyl, F, Cl, Br, OH, OR⁷, NH₂, NHR⁷ oder N(R⁷)₂,
wobei R⁷ ausgewählt ist aus C₁-C₁₀-Alkyl und C₅-C₁₀-Aryl;
- Y¹, Y², Y³: sind unabhängig voneinander eine Bindung, unsubstituiertes oder zumindest monosubstituiertes Methylen, Ethylen, Trimethylen, Tetramethylen, Pentamethylen oder Hexamethylen,
wobei die Substituenten ausgewählt sind aus der Gruppe bestehend aus: F, Cl, Br, OH, OR⁷, CN, NH₂, NHR⁷, N(R⁷)₂ und C₁-C₁₀-Alkyl,
wobei R⁷ ausgewählt ist aus C₁-C₁₀-Alkyl und C₅-C₁₀-Aryl.

In einer weiteren bevorzugten Ausführungsform wird das erfindungsgemäße Verfahren in Gegenwart mindestens eines Komplexkatalysators, der mindestens ein Element ausgewählt aus den Gruppen 8 und 9 des Periodensystems sowie mindestens einen Phosphordonorliganden der allgemeinen Formel (V) enthält, durchgeführt, wobei
- R¹, R², R³, R⁴: sind unabhängig voneinander Methyl, Ethyl, Isopropyl, tert.-Butyl, Cyclopentyl, Cyclohexyl, Phenyl, oder Mesityl;
- A: ist
i) eine Brückengruppe ausgewählt aus der Gruppe Methan, Ethan, Propan, Butan, Cyclohexan, Benzol, Napthalin und Anthracen:
   oder
ii) eine Brückengruppe der Formel (VII) oder (VIII): 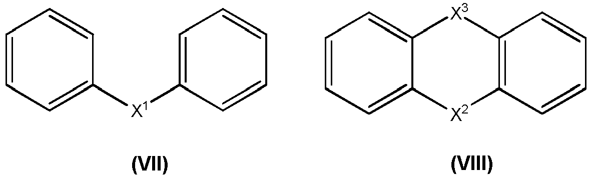
   X¹, X² sind unabhängig voneinander NH, O oder S;
   X³ ist eine Bindung, NH, O, S oder CR¹¹R¹²;
   R¹¹, R¹² sind unabhängig voneinander unsubstituiertes C₁-C₁₀-Alkyl;
- Y¹, Y²: sind unabhängig voneinander eine Bindung, Methylen oder Ethylen.

In einer besonders bevorzugten Ausführungsform wird das erfindungsgemäße Verfahren in Gegenwart mindestens eines Komplexkatalysators, der mindestens ein Element ausgewählt aus den Gruppen 8 und 9 des Periodensystems sowie mindestens einen Phosphordonorliganden der allgemeinen Formel (IX) oder (X) enthält, 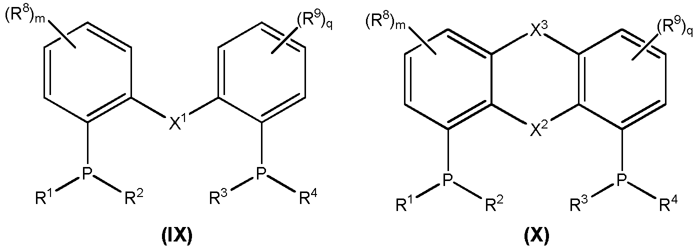 durchgeführt, wobei für m, q, R¹, R², R³, R⁴, R⁸, R⁹, X¹, X² und X³ die vorstehend aufgeführten Definitionen und Bevorzugungen gelten.

In einer weiteren besonders bevorzugten Ausführungsform wird das erfindungsgemäße Verfahren in Gegenwart mindestens eines Komplexkatalysators, der mindestens ein Element ausgewählt aus der Gruppe Ruthenium und Iridium sowie mindestens einen Phosphordonorliganden ausgewählt aus der Gruppe 1,2-Bis(diphenylphosphino)ethan (dppe), 1,2-Bis(dicylohexylphosphino)ethan, 1,3-Bis(diphenylphosphino)propan (dppp), 1,4-Bis(diphenylphosphino)butan (dppb), 2,3-Bis(dicyclohexylphosphino)ethan (dcpe), 4,5-Bis(diphenylphosphino)-9,9-dimethylxanthen (Xantphos), 1,1'-[2,7-Bis(1,1-dimethylethyl)-9,9-dimethyl-9H-xanthen-4,5-diyl]bis[1,1-diphenyl]phosphin (t-Bu-Xantphos), Bis(2-diphenylphosphinoethyl)phenylphosphin und 1,1,1-Tris(Diphenylphosphinomethyl)ethan (Triphos) enthält, durchgeführt. Weiterhin bevorzugt ist der Phosphordonorligand 1,1'-[2,7-bis(1,1-dimethylethyl)-9,9-dimethyl-9H-xanthene-4,5-diyl]bis[1,1-diphenyl]phosphin (t-Bu-Xantphos).

In einer weiteren besonders bevorzugten Ausführungsform wird das erfindungsgemäße Verfahren in Gegenwart eines Komplexkatalysators, der Ruthenium sowie mindestens einen Phosphordonorliganden ausgewählt aus der Gruppe 4,5-Bis(diphenyl-phosphino)-9,9-dimethylxanthen (Xantphos), Bis(2-diphenylphosphinoethyl)phenyl-phosphin und 1,1,1-Tris(Diphenylphosphinomethyl)ethan (Triphos) enthält, durchgeführt.

In einer weiteren besonders bevorzugten Ausführungsform wird das erfindungsgemäße Verfahren in Gegenwart eines Komplexkatalysators, der Iridium sowie mindestens einen Phosphordonorliganden ausgewählt aus der Gruppe 4,5-Bis(diphenylphosphino)-9,9-dimethylxanthen (Xantphos), Bis(2-diphenylphosphinoethyl)phenylphosphin und 1,1,1-Tris(Diphenylphosphinomethyl)ethan (Triphos) enthält, durchgeführt.

Im Rahmen der vorliegenden Erfindung werden unter C₁-C₁₀-Alkyl verzweigte, unverzweigte, gesättigte und ungesättigte Gruppen verstanden. Bevorzugt sind Alkylgruppen mit 1 bis 6 Kohlenstoffatomen (C₁-C₆-Alkyl). Mehr bevorzugt sind Alkylgruppen mit 1 bis 4 Kohlenstoffatomen (C₁-C₄-Alkyl).

Beispiele für gesättigte Alkylgruppen sind Methyl, Ethyl, n-Propyl, Isopropyl, n-Butyl, Isobutyl, sec.-Butyl, tert.-Butyl, Amyl und Hexyl.

Beispiele für ungesättigte Alkylgruppen (Alkenyl, Alkinyl) sind Vinyl, Allyl, Butenyl, Ethinyl und Propinyl.

Die C₁-C₁₀-Alkylgruppe kann unsubstituiert oder, mit einem oder mehreren Substituenten ausgewählt aus der Gruppe F, Cl, Br, Hydroxy (OH), C₁-C₁₀-Alkoxy, C₅-C₁₀-Aryloxy, C₅-C₁₀-Alkylaryloxy, C₅-C₁₀-Heteroaryloxy enthaltend mindestens ein Heteroatom ausgewählt aus N, O, S, Oxo, C₃-C₁₀-Cycloalkyl, Phenyl, C₅-C₁₀-Heteroaryl enthaltend mindestens ein Heteroatom ausgewählt aus N, O, S, C₅-C₁₀-Heterocyclyl enthaltend mindestens ein Heteroatom ausgewählt aus N, O, S, Naphthyl, Amino, C₁-C₁₀-Alkylamino, C₅-C₁₀-Arylamino, C₅-C₁₀-Heteroarylamino enthaltend mindestens ein Heteroatom ausgewählt aus N, O, S, C₁-C₁₀-Dialkylamino, C₁₀-C₁₂-Diarylamino, C₁₀-C₂₀-Alkylarylamino, C₁-C₁₀-Acyl, C₁-C₁₀-Acyloxy, NO₂, C₁-C₁₀-Carboxy, Carbamoyl, Carboxamid, Cyano, Sulfonyl, Sulfonylamino, Sulfinyl, Sulfinylamino, Thiol, C₁-C₁₀-Alkylthiol, C₅-C₁₀-Arylthiol oder C₁-C₁₀-Alkylsulfonyl substituiert sein.
Die vorstehende Definition für C₁-C₁₀-Alkyl gilt für C₁-C₃₀-Alkyl und für C₁-C₆-Alkan sinngemäß.

Unter C₃-C₁₀-Cycloalkyl werden vorliegend gesättigte, ungesättigte monozyklische und polyzyklische Gruppen verstanden. Beispiele für C₃-C₁₀-Cycloalkyl sind Cyclopropyl, Cyclobutyl, Cyclopentyl, Cyclohexyl oder Cycloheptyl. Die Cycloalkylgruppen können unsubstituiert oder substituiert sein mit einem oder mehreren Substituenten, wie sie vorstehend zu der Gruppe C₁-C₁₀-Alkyl definiert wurde.

Die vorstehend genannte Definition für C₃-C₁₀-Cycloalkyl gilt für C₃-C₁₀-Cycloalkan sinngemäß.

Im Rahmen der vorliegenden Erfindung wird unter C₅-C₁₄-Aryl ein aromatisches Ringsystem mit 5 bis 14 Kohlenstoffen verstanden. Das aromatisches Ringsystem kann monozyklisch oder bizyklisch sein. Beispiele für Arylgruppen sind Phenyl, Naphthyl wie 1-Naphthyl und 2-Naphthyl. Die Arylgruppe kann unsubstituiert oder substituiert sein, mit einem oder mehreren Substituenten wie vorstehend unter C₁-C₁₀-Alkyl definiert.

Die vorstehend genannte Definition für C₅-C₁₄-Aryl gilt für C₅-C₁₄-Aromat sinngemäß.

Im Rahmen der vorliegenden Erfindung wird unter C₅-C₁₀-Heteroaryl ein heteroaromatisches System verstanden, das mindestens ein Heteroatom, ausgewählt aus der Gruppe N, O und S, enthält. Die Heteroarylgruppen können monozyklisch oder bizyklisch sein. Für den Fall, dass Stickstoff ein Ringatom ist, umfasst die vorliegende Erfindung auch N-Oxide der stickstoffenthaltenden Heteroaryle. Beispiele für Heteroaryle sind Thienyl, Benzothienyl, 1-Naphthothienyl, Thianthrenyl, Furyl, Benzofuryl, Pyrrolyl, Imidazolyl, Pyrazolyl, Pyridyl, Pyrazinyl, Pyrimidinyl, Pyridazinyl, Indolyl, Isoindolyl, Indazolyl, Purinyl, Isoquinolinyl, Quinolinyl, Acridinyl, Naphthyridinyl, Quinoxalinyl, Quinazolinyl, Cinnolinyl, Piperidinyl, Carbolinyl, Thiazolyl, Oxazolyl, Isothiazolyl, Isoxazolyl. Die Heteroarylgruppen können unsubstituiert oder substituiert sein, mit einem oder mehreren Substituenten, die vorstehend unter C₁-C₁₀-Alkyl definiert wurden.

Die vorstehend genannte Definition für C₅-C₁₀-Heteroaryl gilt für C₅-C₆-Heteroaromat sinngemäß.

Im Rahmen der vorliegenden Erfindung werden unter C₃-C₁₀-Heterocyclyl fünf- bis zehngliedrige Ringsysteme verstanden, die mindestens ein Heteroatom aus der Gruppe N, O und S enthalten. Die Ringsysteme können mono- oder bizyklisch sein. Beispiele für geeignete heterozyklische Ringsysteme sind Piperidinyl, Pyrrolidinyl, Pyrrolinyl, Pyrazolinyl, Pyrazolidinyl, Morpholinyl, Thiomorpholinyl, Pyranyl, Thiopyranyl, Piperazinyl, Indolinyl, Dihydrofuranyl, Tetrahydrofuranyl, Dihydrothiophenyl, Tetrahydrothiophenyl, Dihydropyranyl und Tetrahydropyranyl.

Die vorstehend genannte Definition für C₃-C₁₀-Hetercyclyl gilt für C₃-C₁₀- Heterocycloalkan sinngemäß.

### Alkoholaminierung

Die homogenen Katalysatoren können sowohl direkt in ihrer aktiven Form als auch ausgehend von üblichen Vorstufen unter Zugabe der entsprechenden Liganden erst unter den Reaktionsbedingungen erzeugt werden. Übliche Vorstufen sind beispielsweise [Ru(p-cymene)Cl₂]₂, [Ru(benzol)Cl₂]ₙ, [Ru(CO)₂Cl₂]ₙ, [Ru(CO)₃Cl₂]₂ [Ru(COD)(allyl)], [RuCl₃*H₂O], [Ru(acteylacetonat)₃], [Ru(DMSO)₄Cl₂], [Ru(PPh₃)₃(CO)(H)Cl], [Ru(PPh₃)₃(CO)Cl₂], [Ru(PPh₃)₃(CO)(H)₂], [Ru(PPh₃)₃Cl₂], [Ru(cyclopentadienyl)(PPh₃)₂Cl], [Ru(cyclopentadienyl)(CO)₂Cl], [Ru(cyclopentadienyl)(CO)₂H], [Ru(cyclopentadienyl)(CO)₂]₂, [Ru(pentamethylcyclopentadienyl)(CO)₂Cl], [Ru(pentamethylcylcopentadienyl)(CO)₂H], [Ru(pentamethylcyclopentadienyl)(CO)₂]₂, [Ru(indenyl)(CO)₂Cl], [Ru(indenyl)(CO)₂H], [Ru(indenyl)(CO)₂]₂, Ruthenocen, [Ru(binap)Cl₂], [Ru(bipyridin)₂Cl₂*2H₂O], [Ru(COD)Cl₂]₂, [Ru(pentamethylcyclopentadienyl)(COD)CI], [Ru₃(CO)₁₂], [Ru(tetraphenylhydroxy-cyclopentadienyl)(CO)₂H], [Ru(PMe₃)₄(H)₂], [Ru(PEt₃)₄(H)₂], [Ru(P*n*Pr₃)₄(H)₂], [Ru(P*n*Bu₃)₄(H)₂], [Ru(P*n*Octyl₃)₄(H)₂], [IrCl₃*H₂O], KIrCl₄, K₃IrCl₆, [Ir(COD)Cl]₂, [Ir(cycloocten)₂Cl]₂, [Ir(ethen)₂Cl]₂, [Ir(cyclopentadienyl)Cl₂]₂, [Ir(pentamethylcyclopentadienyl)Cl₂]₂, [Ir(cylopentadienyl)(CO)₂], [Ir(pentamethylcyclopentadienyl)(CO)₂], [Ir(PPh₃)₂(CO)(H)], [Ir(PPh₃)₂(CO)(Cl)], [Ir(PPh₃)₃(Cl)].

Unter homogen-katalysiert wird im Rahmen der vorliegenden Erfindung verstanden, dass der katalytisch-aktive Teil des Komplexkatalysators zumindest teilweise im flüssigen Reaktionsmedium gelöst vorliegt. In einer bevorzugten Ausführungsform liegen mindestens 90 % des im Verfahren eingesetzten Komplexkatalysators im flüssigen Reaktionsmedium gelöst vor, mehr bevorzugt mindestens 95 %, insbesondere bevorzugt mehr als 99 %, am meisten bevorzugt liegt der Komplexkatalysator vollständig gelöst im flüssigen Reaktionsmedium vor (100%), jeweils bezogen auf die Gesamtmenge im flüssigen Reaktionsmedium.

Die Menge der Metallkomponente des Katalysators, bevorzugt Ruthenium oder Iridium, beträgt im Allgemeinen 0,1 bis 5000 Gewichts-ppm, jeweils bezogen auf das gesamte flüssige Reaktionsmedium.

Die Reaktion erfolgt in der Flüssigphase im Allgemeinen bei einer Temperatur von 20 bis 250°C. Bevorzugt wird das erfindungsgemäße Verfahren bei Temperaturen im Bereich von 100 °C bis 200 °C, besonders bevorzugt im Bereich von 110 bis 160°C durchgeführt.

Die Reaktion kann im Allgemeinen bei einem Gesamtdruck von 0,1 bis 20 MPa absolut, der sowohl der Eigendruck des Lösungsmittels bei der Reaktionstemperatur als auch der Druck eines Gases wie Stickstoff, Argon oder Wasserstoff sein kann, durchgeführt werden. Bevorzugt wird das erfindungsgemäße Verfahren bei einem Gesamtdruck im Bereich von 0,5 bis 15 MPa absolut, insbesondere bevorzugt bei einem Gesamtdruck im Bereich von 1 bis 10 MPa absolut durchgeführt.

Die mittlere Reaktionszeit beträgt im Allgemeinen 15 Minuten bis 100 Stunden.

Das Aminierungsmittel (Ammoniak) kann bezüglich der zu aminierenden Hydroxylgruppen in stöchiometrischen, unterstöchiometrischen oder überstöchiometrischen Mengen eingesetzt werden.

In einer bevorzugten Ausführungsform wird Ammoniak mit einem 1,5- bis 250-fachen, bevorzugt mit einem 2- bis 100-fachen, insbesondere mit einem 2- bis 10-fachen molaren Überschuss pro Mol im Edukt umzusetzender Hydroxylgruppen eingesetzt. Es sind auch höhere Überschüsse an Ammoniak möglich.

Das erfindungsgemäße Verfahren kann sowohl in einem Lösungsmittel als auch ohne Lösungsmittel durchgeführt werden. Als Lösungsmittel eignen sich polare und unpolare Lösungsmittel, die in Reinform oder in Mischungen eingesetzt werden können. Beispielsweise kann im erfindungsgemäßen Verfahren nur ein unpolares oder ein polares Lösungsmittel eingesetzt werden. Es ist auch möglich, Mischungen von zwei oder mehr polaren Lösungsmitteln oder Mischungen von zwei oder mehr unpolaren Lösungsmitteln oder Mischungen aus einem oder mehr polaren mit einem oder mehr unpolaren Lösungsmitteln einzusetzen. Auch das Produkt kann als Lösungsmittel in Reinform oder in Mischungen mit polaren oder mit unpolaren Lösungsmittel eingesetzt werden.

Als unpolare Lösungsmittel eignen sich beispielsweise gesättigte und ungesättigte Kohlenwasserstoffe wie Hexan, Heptan, Oktan, Cyclohexan, Benzol, Toluol, Xylol und Mesitylen, und lineare und zyklische Ether wie THF, Diethylether, 1,4-Dioxan, MTBE (tert-Butylmethylether), Diglyme und 1,2-Dimethoxyethan. Bevorzugt werden Toluol, Xylole oder Mesitylen eingesetzt. Je nach Polarität des Produkts, kann auch das Produkt als unpolares Lösungsmittel für die Reaktion eingesetzt werden.

Als polare Lösungsmittel eignen sich beispielsweise Wasser, Dimethylformamid, Formamid, tert-Amylalkohol und Acetonitril. Bevorzugt wird Wasser eingesetzt. Das Wasser kann sowohl vor der Reaktion zugesetzt werden, bei der Reaktion als Reaktionswasser entstehen oder auch nach der Reaktion zusätzlich zum Reaktionswasser zugesetzt werden. Je nach Polarität des Produkts, kann auch das Produkt als polares Lösungsmittel für die Reaktion eingesetzt werden. Ein weiteres bevorzugtes Lösungsmittel ist tert-Amylalkohol.

Für die Umsetzung in der Flüssigphase leitet man Ammoniak, das mindestens eine funktionelle Gruppe der Formel (-CH₂-OH) aufweisende Edukt, gegebenenfalls zusammen mit einem oder mehreren Lösungsmitteln, zusammen mit dem Komplexkatalysator in einen Reaktor.

Die Zuleitung von Ammoniak, Edukt, gegebenenfalls Lösungsmittel und Komplexkatalysator kann dabei simultan oder getrennt voneinander erfolgen. Die Reaktion kann dabei kontinuierlich, in semibatch-Fahrweise, in batch-Fahrweise, rückvermischt in Produkt als Lösungsmittel oder im geraden Durchgang nicht rückvermischt durchgeführt werden.

Für das erfindungsgemäße Verfahren können prinzipiell alle Reaktoren eingesetzt werden, welche für gas/flüssig-Reaktionen unter der gegebenen Temperatur und dem gegebenen Drucks grundsätzlich geeignet sind. Geeignete Standardreaktoren für gas/flüssig- und für flüssig/flüssig-Reaktionssyteme sind beispielsweise in K.D. Henkel, "Reactor Types and Their Industrial Applications", in Ullmann's Encyclopedia of Industrial Chemistry, 2005, Wiley-VCH Verlag GmbH & Co. KGaA, DOI: 10.1002/14356007.b04_087, Kapitel 3.3 "Reactors for gas-liquid reactions" angegeben. Als Beispiele seien genannt Rührkesselreaktoren, Rohrreaktoren oder Blasensäulenreaktoren.

Bei der Aminierungsreaktion wird mindestens eine primäre Hydroxylgruppe (-CH₂-OH) des Edukts mit Ammoniak zu einer primären Aminogruppe (-CH₂-NH₂) umgesetzt, wobei sich jeweils ein Mol Reaktionswasser pro Mol umgesetzter Hydroxylgruppe bildet.

So bilden sich bei der Umsetzung von Alkanolaminen, die nur eine primäre Hydroxylgruppe (-CH₂-OH) aufweisen, die entsprechenden Diamine. Die Umsetzung von Monoaminoethanol führt somit zum entsprechenden 1,2-Diaminoethan.

Bei der Reaktion von Edukten, die neben der funktionellen Gruppe der Formel (-CH₂-OH) eine weitere Hydroxylgruppe aufweisen (Diole), wird bevorzugt nur die primäre Alkoholgruppe (-CH₂-OH) aminiert. Die Umsetzung von 1,2-Ethylenglykol führt somit zum entsprechenden Monoethanolamin. Es ist auch möglich, beide Hydroxylgruppen unter Erhalt von 1,2-Diaminoethan zu aminieren.

Bei der Reaktion von Edukten, die neben der funktionellen Gruppe der Formel (-CH₂-OH) zwei weitere Hydroxylgruppen aufweisen (Triole), wird bevorzugt nur eine primäre Alkoholgruppe (-CH₂-OH) aminiert. Es ist auch möglich zwei oder drei Hydroxylgruppen mit Ammoniak zu den entsprechenden primären Diaminen oder Triaminen umzusetzen. Die Bildung von primären Monoaminen, Diaminen oder Triaminen kann dabei durch die Menge des eingesetzten Ammoniaks und durch die Reaktionsbedingungen gesteuert werden.

Bei der Reaktion von Edukten, die neben der funktionellen Gruppe der Formel (-CH₂-OH) mehr als drei weitere Hydroxylgruppen aufweisen (Polyole), wird bevorzugt nur eine primäre Alkoholgruppe (-CH₂-OH) aminiert. Es ist auch möglich zwei, drei oder mehr Hydroxylgruppen mit Ammoniak zu den entsprechenden primären Monoaminen, Diaminen, Triaminen oder Polyaminen umzusetzen. Die Bildung der primären Monaminen, Diaminen, Triaminen oder Polyaminen kann dabei durch die Menge des eingesetzten Ammoniaks und durch die Reaktionsbedingungen gesteuert werden.

Der bei der Umsetzung entstehende Reaktionsaustrag enthält im Allgemeinen die entsprechenden Aminierungsprodukte, gegebenenfalls das eine oder die mehreren Lösungsmittel, den Komplexkatalysator, gegebenenfalls nicht umgesetzte Edukte und Ammoniak sowie das entstandene Reaktionswasser.

Aus dem Reaktionsaustrag werden der gegebenenfalls vorhandene überschüssige Ammoniak, das gegebenenfalls vorhandene Lösungsmittel, der Komplexkatalysator und das Reaktionswasser entfernt. Das erhaltene Aminierungsprodukt kann weiter aufgearbeitet werden. Der überschüssige Ammoniak, der Komplexkatalysator, gegebenenfalls das oder die Lösungsmittel und gegebenenfalls nicht umgesetzte Edukte können in die Aminierungsreaktion zurückgeführt werden.

Wird die Aminierungsreaktion ohne Lösungsmittel durchgeführt, so ist der homogene Komplexkatalysator nach der Reaktion im Produkt gelöst. Dieser kann im Produkt verbleiben oder durch eine geeignete Methode aus diesem abgetrennt werden. Möglichkeiten zur Abtrennung des Katalysators sind zum Beispiel das Auswaschen mit einem nicht mit dem Produkt mischbaren Lösungsmittel, in welchem sich der Katalysator durch geeignete Wahl der Liganden besser löst als im Produkt. Gegebenenfalls wird der Katalysator durch mehrstufige Extraktion aus dem Produkt abgereichert. Als Extraktionsmittel wird bevorzugt ein auch für die Zielreaktion geeignetes Lösungsmittel wie Toluol, Benzol, Xylole, Alkane, wie Hexane, Heptane und Oktane, und azyklische oder zyklische Ether, wie Diethylether und Tetrahydrofuran eingesetzt, welches nach dem Einengen zusammen mit dem extrahierten Katalysator wieder für die Umsetzung eingesetzt werden kann. Möglich ist auch die Entfernung des Katalysators mit einem geeigneten Absorbermaterial. Eine Abtrennung kann auch durch Zufügen von Wasser zu der Produktphase erfolgen, falls die Reaktion in einem mit Wasser nicht mischbaren Lösungsmittel durchgeführt wird. Löst sich der Katalysator dabei bevorzugt in dem Lösungsmittel, kann er mit dem Lösungsmittel von der wässrigen Produktphase abgetrennt und gegebenenfalls erneut eingesetzt werden. Dies kann durch Wahl geeigneter Liganden bewirkt werden. Die resultierenden wässrigen Mono-, Di-, Tri-, oder Polyamine können direkt als technische Aminlösungen eingesetzt werden. Es ist auch möglich, das Aminierungsprodukt durch Destillation vom Katalysator zu trennen.

Wird die Reaktion in einem Lösungsmittels durchgeführt, so kann dieses mit dem Aminierungsprodukt mischbar sein und nach der Reaktion durch Destillation abgetrennt werden. Es können auch Lösungsmittel eingesetzt werden, welche eine Mischungslücke mit den Aminierungsprodukten oder den Edukten aufweisen. Als geeignete Lösungsmittel hierfür seien beispielsweise Toluol, Benzol, Xylole, Alkane, wie Hexane, Heptane und Oktane, und azyklische oder zyklische Ether, wie Diethylether, Tetrahydrofuran und Dioxan genannt. Durch geeignete Wahl der Phosphinliganden löst sich der Katalysator bevorzugt in der Lösungsmittelphase, d.h. in der nicht-Produktenthaltenden Phase. Die Phosphinliganden können auch so gewählt werden, dass sich der Katalysator im Aminierungsprodukt löst. In diesem Fall lässt sich das Aminierungsprodukt durch Destillation vom Katalysator trennen.

Das Lösungsmittel kann auch unter den Reaktionsbedingungen mit den Edukten und dem Produkt mischbar sein und erst nach dem Abkühlen eine zweite flüssige Phase ausbilden, welche den Großteil des Katalysators enthält. Als Lösungsmittel, die diese Eigenschaft zeigen, seien beispielsweise Toluol, Benzol, Xylole, Alkane, wie Hexane, Heptane und Oktane, genannt. Der Katalysator kann dann zusammen mit dem Lösungsmittel abgetrennt und wieder verwendet werden. Die Produktphase kann auch in dieser Variante mit Wasser versetzt werden. Der in dem Produkt enthaltene Teil des Katalysators kann anschließend durch geeignete Absorbermaterialien wie beispielsweise Polyacrylsäure und deren Salze, sulfonierte Polystyrole und deren Salze, Aktivkohlen, Montmorillonite, Bentonite sowie Zeolithe abgetrennt werden oder aber in dem Produkt belassen werden.

Die Aminierungsreaktion kann auch zweiphasig durchgeführt werden. Bei der Ausführungsform der zweiphasigen Reaktionsführung eignen sich als unpolare Lösungsmittel besonders Toluol, Benzol, Xylole, Alkane, wie Hexane, Heptane und Oktane, in Kombination mit lipophilen Phosphinliganden am Übergangsmetallkatalysator, wodurch sich der Übergangsmetallkatalysator in der unpolaren Phase anreichert. Bei dieser Ausführungsform, in welcher das Produkt sowie das Reaktionswasser und ggf. nicht umgesetzte Edukte eine mit diesen Verbindungen angereicherte Zweitphase bilden, kann der Großteil des Katalysators durch einfache Phasentrennung von der Produktphase abgetrennt und wiederverwendet werden.

Falls flüchtige Nebenprodukte oder nicht umgesetzte Edukte oder auch das bei der Reaktion entstandene oder nach der Reaktion zur besseren Extraktion zugesetzte Wasser unerwünscht sind, können diese problemlos von dem Produkt durch Destillation abgetrennt werden.

Es kann auch vorteilhaft sein, das bei der Reaktion gebildete Wasser kontinuierlich aus dem Reaktionsgemisch zu entfernen. Das Reaktionswasser kann direkt durch Destillation aus dem Reaktionsgemisch oder als Azeotrop unter Zusatz eines geeigneten Lösungsmittels (Schleppers) und unter Verwendung eines Wasserabscheiders abgetrennt, oder durch Zusatz von Wasser entziehenden Hilfsstoffen entfernt werden.

Der Zusatz von Basen kann einen positiven Effekt auf die Produktbildung haben. Als geeignete Basen seien hier Alkalihydroxide, Erdalkalihydroxide, Alkalialkoholate, Erdalkalialkoholate, Alkali-Carbonate und Erdalkalicarbonate genannt, von welchen 0,01 bis 100 molare Äquivalente in Bezug auf den verwendeten Metallkatalysator eingesetzt werden können.

Ein weiterer Gegenstand der vorliegenden Erfindung ist die Verwendung eines Komplexkatalysators, der mindestens ein Element ausgewählt aus den Gruppen 8 und 9 des Periodensystems sowie mindestens einen Phosphordonorliganden der allgemeine Formel (I) enthält, 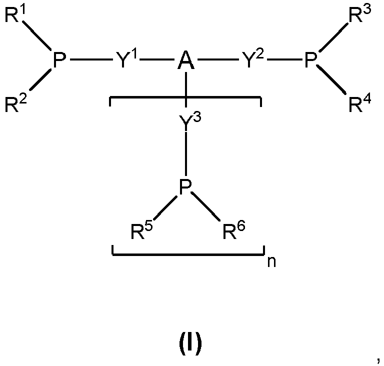 wobei
- n: ist 0 oder 1;
- R¹, R², R³, R⁴, R⁵, R⁶: sind unabhängig voneinander unsubstituiertes oder zumindest monosubstituiertes C₁-C₁₀-Alkyl, C₁-C₄-Alkyldiphenylphosphin (-C₁-C₄-Alkyl-P(Phenyl)₂), C₃-C₁₀-Cycloalkyl, C₃-C₁₀-Heterocyclyl enthaltend mindestens ein Heteroatom ausgewählt aus N, O und S, C₅-C₁₄-Aryl oder C₅-C₁₀-Heteroaryl enthaltend mindestens ein Heteroatom ausgewählt aus N, O und S,
wobei die Substituenten ausgewählt sind aus der Gruppe bestehend aus: F, Cl, Br, OH, CN, NH₂ und C₁-C₁₀-Alkyl;
- A: ist
i) eine Brückengruppe ausgewählt aus der Gruppe unsubstituiertes oder zumindest monosubstituiertes N, O, P, C₁-C₆-Alkan, C₃-C₁₀-Cycloalkan, C₃-C₁₀-Heterocycloalkan enthaltend mindestens ein Heteroatom ausgewählt aus N, O und S, C₅-C₁₄-Aromat oder C₅-C₆-Heteroaromat enthaltend mindestens ein Heteroatom ausgewählt aus N, O und S,
   wobei die Substituenten ausgewählt sind aus der Gruppe bestehend aus: C₁-C₄-Alkyl, Phenyl, F, Cl, Br, OH, OR⁷, NH₂, NHR⁷ oder N(R⁷)₂,
   wobei R⁷ ausgewählt ist aus C₁-C₁₀-Alkyl und C₅-C₁₀-Aryl;
   oder
ii) eine Brückengruppe der Formel II oder III: 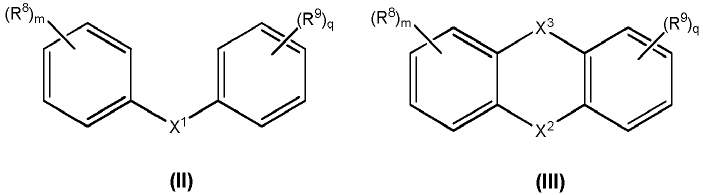
   m, q sind unabhängig voneinander 0, 1, 2, 3 oder 4;
   R⁸, R⁹ sind unabhängig voneinander ausgewählt aus der Gruppe C₁-C₁₀-Alkyl, F, Cl, Br, OH, OR⁷, NH₂, NHR⁷ und N(R⁷)₂,
   wobei R⁷ ausgewählt ist aus C₁-C₁₀-Alkyl und C₅-C₁₀-Aryl;
   X¹, X² sind unabhängig voneinander NH, O oder S;
   X³ ist eine Bindung, NH, NR¹⁰, O, S oder CR¹¹R¹²;
   R¹⁰ ist unsubstituiertes oder zumindest monosubstituiertes C₁-C₁₀-Alkyl, C₃-C₁₀-Cycloalkyl, C₃-C₁₀-Heterocyclyl enthaltend mindestens ein Heteroatom ausgewählt aus N, O und S, C₅-C₁₄-Aryl oder C₅-C₁₀-Heteroaryl enthaltend mindestens ein Heteroatom ausgewählt aus N, O und S,
   wobei die Substituenten ausgewählt sind aus der Gruppe bestehend aus: F, Cl, Br, OH, CN, NH₂ und C₁-C₁₀-Alkyl;
   R¹¹, R¹² sind unabhängig voneinander unsubstituiertes oder zumindest monosubstituiertes C₁-C₁₀-Alkyl, C₁-C₁₀-Alkoxy, C₃-C₁₀-Cycloalkyl, C₃-C₁₀-Cycloalkoxy,C₃-C₁₀-Heterocyclyl enthaltend mindestens ein Heteroatom ausgewählt aus N, O und S, C₅-C₁₄-Aryl, C₅-C₁₄-Aryloxy oder C₅-C₁₀-Heteroaryl enthaltend mindestens ein Heteroatom ausgewählt aus N, O und S,
   wobei die Substituenten ausgewählt sind aus der Gruppe bestehend aus: F, Cl, Br, OH, CN, NH₂ und C₁-C₁₀-Alkyl;
- Y¹, Y², Y³: sind unabhängig voneinander eine Bindung, unsubstituiertes oder zumindest monosubstituiertes Methylen, Ethylen, Trimethylen, Tetramethylen, Pentamethylen oder Hexamethylen,
wobei die Substituenten ausgewählt sind aus der Gruppe bestehend aus: F, Cl, Br, OH, OR⁷, CN, NH₂, NHR⁷, N(R⁷)₂ und C₁-C₁₀-Alkyl,
wobei R⁷ ausgewählt ist aus C₁-C₁₀-Alkyl und C₅-C₁₀-Aryl,
zur homogen-katalysierten Herstellung von primären Aminen, die mindestens eine funktionelle Gruppe der Formel (-CH₂-NH₂) aufweisen, durch Alkoholaminierung von Edukten, die mindestens eine funktionelle Gruppe der Formel (-CH₂-OH) aufweisen, mit Ammoniak.

Für die Verwendung des Komplexkatalysators zur homogen-katalysierten Herstellung von primären Aminen, die mindestens eine funktionelle Gruppe der Formel (-CH₂-NH₂) aufweisen, durch Alkoholaminierung von Edukten, die mindestens eine funktionelle Gruppe der Formel (-CH₂-OH) aufweisen, mit Ammoniak gelten die für das erfindungsgemäße Verfahren beschriebenen Definitionen und Bevorzugungen.

Die Erfindung wird durch die nachfolgenden Beispiele verdeutlicht, ohne sie darauf zu beschränken.

### Beispiele

Generelle Vorschrift für die erfindungsgemäße katalytische Aminierung von Alkoholen mit Ammoniak:
Ligand L, Metallsalz M, Lösungsmittel und der angegebene Alkohol wurden unter Ar-Atmosphäre in einem 160 ml Parr-Autoklaven (Firma hte, (Edelstahl V4A)) mit magnetisch gekoppeltem Schrägblattrührer (Rührgeschwindigkeit: 200-500 Umdrehungen/Minute) vorgelegt. Die angegebene Menge an Ammoniak wurde bei Raumtemperatur entweder vorkondensiert oder direkt aus der NH₃-Druckgasflasche zudosiert. Falls Wasserstoff eingesetzt wurde, erfolgte dies durch iterative Differenzdruck-Dosierung. Der Stahlautoklav wurde auf die angegebene Temperatur elektrisch aufgeheizt und für die angegebene Zeit unter Rühren (500 Umdrehungen/Minute) erhitzt (Innentemperaturmessung). Nach dem Abkühlen auf Raumtemperatur, Entspannung des Autoklaven und Ausgasen des Ammoniaks bei Normaldruck wurde die Reaktionsmischung mittels GC (30m RTX5 Amin 0,32mm 1,5µm) analysiert. Eine Aufreinigung der jeweiligen Produkte kann z.B. durch Destillation erfolgen. Die Ergebnisse für die Aminierung von Octanol (Tabellen 1 a und 1 b), 1,4-Butandiol (Tabelle 2), Diethylenglykol (Tabelle 3), 1,9-Nonandiol, 1,6-Hexandiol, 1,10-Decandiol (Tabelle 4) und 1,2-Dimethanolfuran (Tabelle 5) sind im Folgenden angegeben:

| Ligandenbezeichnung (L) | CAS | IUPAC |
|---|---|---|
| Triphos | 22031-12-5 | 1,1,1-Tris(diphenylphosphino-methyl)ethan |
| Xantphos | 161265-03-8 | 4,5-Bis(diphenylphosphino)-9,9-dimethylxanthen |
| S-Phos | 657408-07-6 | 2-Dicyclohexylphosphino-2',6'-dimethoxybiphenyl |
| Rhodaphos | 22031-14-7 | 1,1,1-Tris(diethylposphino-methyl)ethan |
| DPPEPP | 23582-02-7 | Bis(2-diphenylphosphinoethyl)phenylphosphin |
| Tetraphos | 23582-03-8 | Tris[2-(diphenylphosphino)ethyl]phosphin |
| tBu-Xantphos | 221462-97-1 | 1,1'-[2,7-bis(1,1-dimethylethyl)-9,9-dimethyl-9H-xanthene-4,5-diyl]bis[1,1-diphenyl]phosphin |
| tBuPPyP | 338800-13-8 | 2,6-Bis[(di-tert-butylphosphino)methyl]pyridin |
| DPEPhos | 166330-10-5 | Bis[2-(diphenylphosphanyl)phenyl]ether |
| Depe | 6411-21-8 | 1,2-Bis(diethylphosphino)ethane |
| dppb | 7688-25-7 | 1,4-Bis(diphenylphosphino)butane |

**Tabelle 1a**

| | | | | | | | | | | |
|---|---|---|---|---|---|---|---|---|---|---|
| 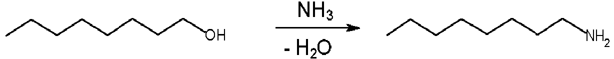 | | | | | | | | | | |

| **Nr^{a)}** | **Lösungsmittel** | **T[°C]** | **NH₃ [Eq.]^{f)}** | **Reaktionsdruck [bar]** | **Metallsalz [M]** | **Met. [M] (mol%)** | **Ligand [L]** | **Lig. [L] (mol%)** | **Umsatz ^{b)}** | **Selektivität ^{c)}** |
|---|---|---|---|---|---|---|---|---|---|---|
| 1 | p-Xylol | 155 | 6 | 42 | [RuHCl(CO)(PPh₃)₃] | 0,10 | | | 6,1 | 1,4 |
| 2 | p-Xylol | 155 | 6 | 41 | [RuHCl(CO)(PPh₃)₃] | 0,10 | Triphos | 0,10 | 58,6 | 83,6 |
| 3 | Toluol | 180 | 6 | 40 | [RuHCl(CO)(PPh₃)₃] | 0,20 | Triphos | 0,20 | 99,1 | 91,8 |
| 5 | p-Xylol | 155 | 6 | 43 | [Ru(COD)methylallyl₂] | 0,10 | Tetraphos | 0,10 | 13,1 | 21,4 |
| 6 | p-Xylol | 155 | 6 | 43 | [RuHCl(CO)(PPh₃)₃] | 0,10 | Xantphos | 0,10 | 29,8 | 77,1 |
| 7 | p-Xylol | 155 | 6 | 42 | [RuHCl(CO)(PPh₃)₃] | 0,10 | Triphenylphos phin | 0,30 | 6,4 | 0,4 |
| 8 | p-Xylol | 155 | 6 | 41 | [Ru(COD)methylallyl₂] | 0,10 | Sphos | 0,10 | 3,5 | 21,7 |
| 9 | Toluol | 155 | 6 | 42 | [RuHCl(CO)(PPh₃)₃] | 0,10 | DPPEPP | 0,10 | 46,9 | 74,4 |
| 10 | Toluol | 155 | 6 | 44 | [RuHCl(CO)(PPh₃)₃] | 0,10 | Rhodaphos | 0,10 | 24,0 | 44,8 |
| 11 ^{d)} | p-Xylol | 160 | 6 | n.b. | [Ru(COD)methylallyl₂] | 0,20 | DPEPhos | 0,20 | 14,7 | 20,0 |
| 12^{d, e)} | p-Xylol | 160 | 6 | n.b. | [Ru(COD)Cl₂] | 0,20 | depe | 0,20 | 16,6 | 29.5 |
| 13^{d)} | p-Xylol | 160 | 6 | n.b. | [Ru(COD)methylallyl₂] | 0,20 | tBuPPyP | 0,20 | 19,5 | 25,2 |
| 15 | Toluol | 155 | 6 | 38 | [Ir(COD)Cl]₂ | 0,10 | Triphos | 0,20 | 2,4 | 1,3 |
| 16 | Toluol | 155 | 6 | 42 | [Ir(COD)Cl]₂ | 0,10 | Xantphos | 0,20 | 11,6 | 48,8 |
| a) 50ml Lösungsmittel; Ansatzgröße: 50mmol Octanol, Reaktionszeit: 12 h; b) Auswertung per GC (FI.%); c) Produktselektivität zu n-Octylamin bestimmt per GC (FI.%) d) 10ml Lösungsmittel; Ansatzgröße: 25mmol Substrat e) Zugabe von 0,4 mol% KOtBu (bezogen auf Octanol); f) Molare Equivalente NH₃ pro OH-Funktion am Substrat; | | | | | | | | | | |

**Tabelle 1 b**

| **Nr^{a)}** | **Lösungsmittel** | **T[°C]** | **NH₃ [Eq.]^{d)}** | **Reaktionsdruck [bar]** | **Metallsalz [M]** | **Met. [M] (mol%)** | **Ligand [L]** | **Lig. [L] (mol%)** | **Umsatz ^{b)}** | **Selektivität ^{c)}** |
|---|---|---|---|---|---|---|---|---|---|---|
| 1 | Toluol | 180 | 6 | 41,8 | [RuHCl(CO)(PPh₃)₃] | 0,10 | DPPEPP | 0,10 | 91,5 | 81,8 |
| 2 | Toluol | 155 | 6 | 43,2 | [Ru(COD)Cl₂] | 0,10 | t-Bu-Xantphos | 0,10 | 18,7 | 67,3 |
| 3^{e)} | Toluol | 155 | 6 | 39,2 | [RuHCl(CO)(PPh₃)₃] | 0,2 | Triphos | 0,2 | 47,0 | 83,8 |
| 4^{f)} | Toluol | 155 | 6 | 42,2 | [RuHCl(CO)(PPh₃)₃] | 0,2 | Triphos | 0,2 | 70,4 | 84,5 |
| 5^{g)} | Toluol | 155 | 6 | 43,0 | [RuHCl(CO)(PPh₃)₃] | 0,2 | Triphos | 0,2 | 50,7 | 85,6 |
| 6^{h)} | Toluol | 155 | 6 | 42,1 | [RuHCl(CO)(PPh₃)₃] | 0,2 | Triphos | 0,2 | 67,2 | 85,9 |
| 7 | THF | 155 | 6 | 39,9 | [RuHCl(CO)(PPh₃)₃] | 0,2 | dppb | 0,2 | 35,2 | 75,2 |
| a) 50ml Lösungsmittel; Ansatzgröße: 50mmol Octanol, Reaktionszeit: 12 h; b) Auswertung per GC (FI.%); c) Produktselektivität zu n-Octylamin bestimmt per GC (FI.%) d) Molare Equivalente NH₃ pro OH-Funktion am Substrat; e) Zugabe von 50mmol H₂O; f) Zugabe von 50mmol Hexylamin; g) Zugabe von 25mmol H₂O; h) Zugabe von 25mmol Hexylamin; | | | | | | | | | | |

**Tabelle 2**

| | | | | | | | | | | | |
|---|---|---|---|---|---|---|---|---|---|---|---|
| 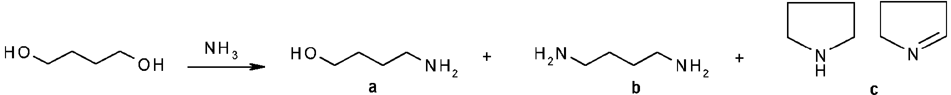 | | | | | | | | | | | |

| **Nr**^{**a**)} | **T [°C)** | **NH₃ [Eq.]^{e)}** | **Reaktions druck [bar]** | **Metallsalz [M]** | **Met. [M] (mol%)^{f)}** | **Ligand [L]** | **Lig. [L]** | **Umsatz ^{b}** | **Selektivität ^{c}** | | |
|---|---|---|---|---|---|---|---|---|---|---|---|
| | | | | | | | **(mol%)^{f)}** | | **a :** | **b :** | **c** |
| 1 | 155 | 6 | 49 | [RuHCl(CO)(PPh₃)₃] | 0,1 | Triphos | 0,1 | 74,7 | 59,1 | 0,7 | 6,7 |
| 2 | 155 | 6 | 66^{d)} | [RuHCl(CO)(PPh₃)₃] | 0,1 | Triphos | 0,1 | 61,8 | 78,0 | 0,6 | 5,4 |
| 3 | 180 | 6 | 49 | [RuHCl(CO)(PPh₃)₃] | 0,2 | Triphos | 0,2 | 99,9 | 1,7 | 4,7 | 37,7 |
| 4 | 155 | 6 | 45 | [RuHCl(CO)(PPh₃)₃] | 0,1 | Xantphos | 0,1 | 35,0 | 81,8 | 0,0 | 6,4 |
| 5 | 155 | 6 | 47 | [Ru(COD)methylallyl₂] | 0,1 | Tetraphos | 0,1 | 6,0 | 8,5 | 0,0 | 1,6 |
| 6 | 155 | 6 | 39 | [RuHCl(CO)(PPh₃)₃] | 0,2 | Rhodaphos | 0,2 | 39,8 | 17,5 | 0,0 | 4,6 |
| 7 | 155 | 6 | 38 | [RuHCl(CO)(PPh₃)₃] | 0,2 | DPPEPP | 0,2 | 66,6 | 68,1 | 0,1 | 11,0 |
| a) 50ml Toluol; Ansatzgröße: 25 mmol 1,4-Butandiol, Reaktionszeit: 12 h; b) Auswertung per GC (FI.%); c) Produktselektivität bestimmt per GC (FI.%); d) kalt aufgepresst: 5 bar H₂, 8 bar NH₃, e) Molare Equivalente NH₃ pro OH-Funktion am Substrat, f) mol% bezogen auf Anzahl der OH-Funktionen am Substrat; | | | | | | | | | | | |

**Tabelle 3**

| | | | | | | | | | | | |
|---|---|---|---|---|---|---|---|---|---|---|---|
| 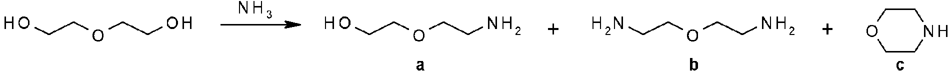 | | | | | | | | | | | |

| **Nr**^{**a**)} | **T[°C]** | **NH₃** [**Eq**.]^{**e**)} | **Reaktionsdruck [bar]** | **Metallsalz [M]** | **Met. [M] (mol%)^{f)}** | **Ligand [L]** | **Lig. [L] (mol%)^{f)}** | **Umsatz b** | **Selektivität^{c}** | | |
|---|---|---|---|---|---|---|---|---|---|---|---|
| | | | | | | | | | **a :** | **b :** | **c** |
| 1 | 155 | 6 | 41 | [RuHCl(CO)(PPh₃)₃] | 0,1 | Triphos | 0,1 | 51,0 | 66,2 | 0,9 | 5,9 |
| 2 | 155 | 6 | 59^{d)} | [RuHCl(CO)(PPh₃)₃] | 0,1 | Triphos | 0,1 | 16,2 | 87,3 | 0,1 | 2,3 |
| 3 | 180 | 6 | 41 | [RuHCl(CO)(PPh₃)₃] | 0,2 | Triphos | 0,2 | 97,6 | 26,4 | 13,4 | 54,0 |
| 4 | 180 | 6 | 43 | [RuHCl(CO)(PPh₃)₃] | 0,2 | Xantphos | 0,1 | 27,7 | 67,1 | 0,2 | 5,3 |
| 5 | 155 | 6 | 44 | [Ru(COD)methylallyl₂] | 0,1 | Tetraphos | 0,1 | 3,9 | 0,0 | 0,0 | 1,1 |
| 6 | 155 | 6 | 40 | [RuHCl(CO)(PPh₃)₃] | 0,2 | Rhodaphos | 0,2 | 21,8 | 4,8 | 0,0 | 1,3 |
| 7 | 155 | 6 | 38 | [RuHCl(CO)(PPh₃)₃] | 0,2 | DPPEPP | 0,2 | 21,5 | 46,0 | 0,0 | 1,8 |
| a) 50ml Toluol; Ansatzgröße: 25 mmol Diethylenglykol, Reaktionszeit: 12 h; b) Auswertung per GC (FI.%); c) Produktselektivität bestimmt per GC (FI.%); d) kalt aufgepresst: 5 bar H₂, 8 bar NH₃, e) Molare Äquivalente NH₃ pro OH-Funktion am Substrat, f) mol% bezogen auf Anzahl der OH-Funktionen am Substrat | | | | | | | | | | | |

**Tabelle 4**

| | | | | | | | | | | | | | |
|---|---|---|---|---|---|---|---|---|---|---|---|---|---|
| 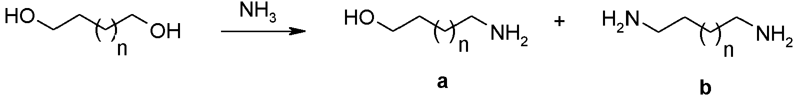 | | | | | | | | | | | | | |

| **Nr^{a)}** | **Substrat** | **T [°C]** | **Reaktionsze it [t]** | **NH₃ [Eq.]^{e)}** | **Reaktionsdruck [bar]** | **Lösungsmittel (wasserfrei)** | **Metallsalz [M]** | **Met. [M] (mol%) ^{f)}** | **Ligand [L]** | **Lig. [L] (mol%) ^{f)}** | **Umsatz ^{b)}** | **Selektivität ^{c}** | |
|---|---|---|---|---|---|---|---|---|---|---|---|---|---|
| | | | | | | | | | | | | **a** | **: b** |
| 1 | 1,6-Hexandiol | 155 | 12 | 6 | 42 | Toluol | [RuHCl(CO)(PPh₃)₃] | 0,10 | Triphos | 0,10 | 83,0 | 61,3 | 25,7 |
| 2 | 1,6-Hexandiol | 155 | 12 | 6 | 36 | Toluol | [RuHCl(CO)(PPh₃)₃] | 0,10 | Xantphos | 0,10 | 33,4 | 84,9 | 4,6 |
| 3 | 1,6-Hexandiol | 155 | 12 | 6 | 40 | Toluol | [RuHCl(CO)(PPh₃)₃] | 0,10 | DPPEPP | 0,10 | 70,7 | 66,5 | 16,0 |
| 4 | 1,6-Hexandiol | 155 | 12 | 6 | 44 | Toluol | [RuHCl(CO)(PPh₃)₃] | 0,10 | Rhodaphos | 0,10 | 35,1 | 53,0 | 2,0 |
| 5 | 1,10-Decandiol | 155 | 24 | 6 | 39 | Toluol | [RuHCl(CO)(PPh₃)₃] | 0,20 | Triphos | 0,20 | 85,7 | 43,0 | 44,4 |
| 6 | 1,10-Decandiol | 180 | 24 | 6 | 43 | Toluol | [RuHCl(CO)(PPh₃)₃] | 0,20 | Triphos | 0,20 | 93,3 | 2,0 | 90,1 |
| 7^{d)} | 1,9-Nonandiol | 155 | 24 | 12 | 14 | Mesitylen | [RuHCl(CO)(PPh₃)₃] | 0,20 | Triphos | 0,20 | 79,3 | 54,0 | 31,1 |
| a) 50ml Lösungsmittel; Ansatzgröße: 25 mmol Diol; b) Auswertung per GC (FI.%); c) Produktselektivität bestimmt per GC (FI.%); d) Ansatzgröße: 50 mmol Substrat; e) Molare Equivalente NH₃ pro OH-Funktion am Substrat; f) mol% bezogen auf Anzahl der OH-Funktionen am Substrat | | | | | | | | | | | | | |

**Tabelle 5**

| | | | | | | | | | | | | | | |
|---|---|---|---|---|---|---|---|---|---|---|---|---|---|---|
| 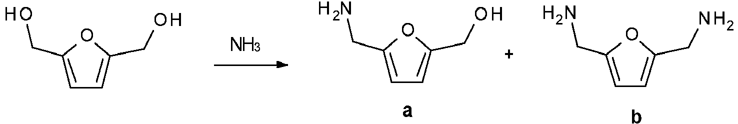 | | | | | | | | | | | | | | |

| **Nr^{a)}** | **Substrat** | **T [°C]** | **Reaktionszeit [t]** | **konz. [mol/l]** | **NH₃ [Eq.]^{d)}** | **Reaktionsdruck [bar]** | **Lösungsmittel (wasserfrei)** | **Metallsalz [M]** | **Met. [M] (mol%) e)** | **Ligand [L]** | **Lig. [L] (mol%)^{e)}** | **Umsatz ^{b)}** | **Selektivität ^{c)}** | |
|---|---|---|---|---|---|---|---|---|---|---|---|---|---|---|
| | | | | | | | | | | | | | **a** | **: b** |
| 1 | 2,5-Dimethanolfuran | 140 | 24 | 1 | 6 | 15 | THF | [RuHCl(CO)(PPh₃)₃] | 0,20 | Triphos | 0,20 | 46,8 | 63,1 | 10,2 |
| a) 40ml Lösungsmittel; Ansatzgröße: 40 mmol Diol; b) Auswertung per GC (FI.%); c) Produktselektivität bestimmt per GC (FI.%); d) Molare Äquivalente NH₃ pro OH-Funktion am Substrat; e) mol% bezogen auf Anzahl der OH-Funktionen am Substrat; | | | | | | | | | | | | | | |

## Patentansprüche

1. Verfahren zur Herstellung von primären Aminen, die mindestens eine funktionelle Gruppe der Formel (-CH₂-NH₂) aufweisen, durch Alkoholaminierung von Edukten, die mindestens eine funktionelle Gruppe der Formel (-CH₂-OH) aufweisen, mit Ammoniak unter Wasserabspaltung, wobei die Alkoholaminierung homogen-katalysiert in Gegenwart mindestens eines Komplexkatalysators, der mindestens ein Element ausgewählt aus den Gruppen 8 und 9 des Periodensystems sowie mindestens einen Phosphordonorliganden der allgemeinen Formel (I) enthält, 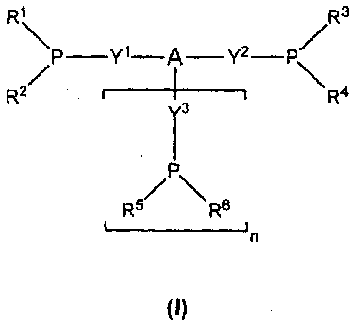 durchgeführt wird, wobei
n ist 0 oder 1,
R¹, R², R³, R⁴, R⁵, R⁸ sind unabhängig voneinander unsubstituiertes oder zumindest monosubstituiertes C₁-C₁₀-Alkyl, C₁-C₄-Alkyldiphenylphosphin (-C₁-C₄-Alkyl-P(Phenyl)₂), C₃-C₁₀-Cycloalkyl, C₃-C₁₀-Heterocyclyl enthaltend mindestens ein Heteroatom ausgewählt aus N, O und S, C₅-C₁₄-Aryl oder C₅-C₁₀-Heteroaryl enthaltend mindestens ein Heteroatom ausgewählt aus N, O und S,
wobei die Substituenten ausgewählt sind aus der Gruppe bestehend aus: F, Cl, Br, OH, CN, NH₂ und C₁-C₁₀-Alkyl;
A ist
i) eine Brückengruppe ausgewählt aus der Gruppe unsubstituiertes oder zumindest monosubstituiertes N, O, P, C₁-C₈-Alkan, C₃-C₁₀-Cycloalkan, C₃-C₁₀-Heterocycloalkan enthaltend mindestens ein Heteroatom ausgewählt aus N, O und S, C₅-C₆-Heteroaromat enthaltend mindestens ein Heteroatom ausgewählt aus N, O und S und C₅-C₁₄-Aromat,
wobei die Substituenten ausgewählt sind aus der Gruppe bestehend aus: C₁-C₄-Alkyl, Phenyl, F, Cl, Br, OH, OR⁷, NH₂, NHR⁷ oder N(R⁷)₂,
wobei R⁷ ausgewählt ist aus C₁-C₁₀-Alkyl und C₆-C₁₀-Aryl;
oder
ii) eine Brückengruppe der Formel (II) oder (III): 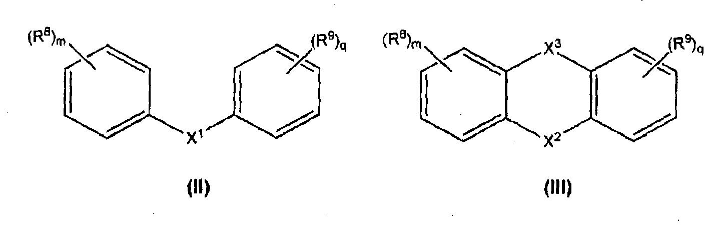
m, q sind unabhängig voneinander 0, 1, 2, 3 oder 4,
R⁸, R⁹ sind unabhängig voneinander ausgewählt aus der Gruppe C₁-C₁₀-Alkyl, F, Cl, Br, OH, OR⁷, NH₂, NHR⁷ und N(R⁷)₂,
wobei R⁷ ausgewählt ist aus C₁-C₁₀-Alkyl und C₅-C₁₀-Aryl;
X¹, X² sind unabhängig voneinander NH, O oder S,
X³ ist eine Bindung, NH, NR¹⁰, O, S oder CR¹¹R¹²,
R¹⁰ ist unsubstituiertes oder zumindest monosubstituiertes C₁-C₁₀-Alkyl, C₃-C₁₀-Cycloalkyl, C₃-C₁₀-Heterocyclyl enthaltend mindestens ein Heteroatom ausgewählt aus N, O und S, C₅-C₁₄-Aryl oder C₅-C₁₀-Heteroaryl enthaltend mindestens ein Heteroatom ausgewählt aus N, O und S,
wobei die Substituenten ausgewählt sind aus der Gruppe bestehend aus: F, Cl, Br, OH, CN, NH₂ und C₁-C₁₀-Alkyl;
R¹¹, R¹² sind unabhängig voneinander unsubstituiertes oder zumindest monosubstituiertes C₁-C₁₀-Alkyl, C₁-C₁₀-Alkoxy, C₃-C₁₀-Cycloalkyl, C₃-C₁₀-Cycloalkoxy, C₃-C₁₀-Heterocyclyl enthaltend mindestens ein Heteroatom ausgewählt aus N, O und S, C₅-C₁₄-Aryl, C₅-C₁₄-Aryloxy oder C₅-C₁₀-Heteroaryl enthaltend mindestens ein Heteroatom ausgewählt aus N, O und S,
wobei die Substituenten ausgewählt sind aus der Gruppe bestehend aus: F, Cl, Br, OH, CN, NH₂ und C₁-C₁₀-Alkyl;
Y¹, Y², Y³ sind unabhängig voneinander eine Bindung, unsubstituiertes oder zumindest monosubstituiertes Methylen, Ethylen, Trimethylen, Tetramethylen, Pentamethylen oder Hexamethylen,
wobei die Substituenten ausgewählt sind aus der Gruppe bestehend aus: F, Cl, Br, OH, OR⁷, CN, NH₂, NHR⁷, N(R⁷)₂ und C₁-C₁₀-Alkyl,
wobei R⁷ ausgewählt ist aus C₁-C₁₀-Alkyl und C₅-C₁₀-Aryl.

2. Verfahren nach Anspruch 1, wobei
n ist 0 oder 1;
R¹, R², R³, R⁴, R⁵, R⁶ sind unabhängig voneinander unsubstituiertes C₁-C₁₀-Alkyl, C₁-C₄-Alkyldiphenylphosphin, C₃-C₁₀-Cycloalkyl, C₃-C₁₀-Heterocyclyl enthaltend mindestens ein Heteroatom ausgewählt aus N, O und S, C₅-C₁₄-Aryl oder C₅-C₁₀-Heteroaryl enthaltend mindestens ein Heteroatom ausgewählt aus N, O und S;
A ist
i) eine Brückengruppe ausgewählt aus der Gruppe unsubstituiertes N, O, P, C₁-C₆-Alkan, C₃-C₁₀-Cycloalkan, C₃-C₁₀-Heterocycloalkan enthaltend mindestens ein Heteroatom ausgewählt aus N, O und S, C₅-C₆-Heteroaromat enthaltend mindestens ein Heteroatom ausgewählt aus N, O und S und C₅-C₁₄-Aromat;
oder
ii) eine Brückengruppe der Formel II oder III: 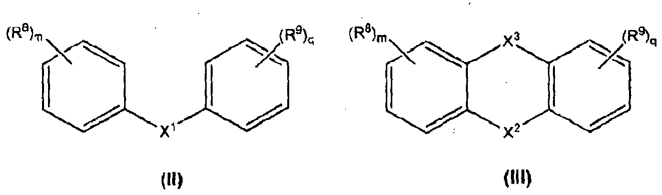
m, q sind unabhängig voneinander 0, 1, 2, 3 oder 4;
R⁸, R⁹ sind unabhängig voneinander ausgewählt aus der Gruppe C₁-C₁₀-Alkyl, F, Cl, Br, OH, OR⁷, NH₂, NHR⁷ und N(R⁷)₂,
wobei R⁷ ausgewählt ist aus C₁-C₁₀-Alkyl und C₅-C₁₀-Aryl;
X¹, X² sind unabhängig voneinander NH, O oder S;
X³ ist eine Bindung, NH, NR¹⁰, O, S oder CR¹¹R¹²;
R¹⁰ ist unsubstituiertes C₁-C₁₀-Alkyl, C₃-C₁₀-Cycloalkyl, C₃-C₁₀-Heterocyclyl enthaltend mindestens ein Heteroatom ausgewählt aus N, O und S, C₆-C₁₄-Aryl oder C₅-C₁₀-Heteroaryl enthaltend mindestens ein Heteroatom ausgewählt aus N, O und S;
R¹¹, R¹² sind unabhängig voneinander unsubstituiertes C₁-C₁₀-Alkyl, C₁-C₁₀-Alkoxy, C₃-C₁₀-Cycloalkyl, C₃-C₁₀-Cycloalkoxy,C₃-C₁₀-Heterocyclyl enthaltend mindestens ein Heteroatom ausgewählt aus N, O und S, C₅-C₁₄-Aryl, C₅-C₁₄-Aryloxy oder C₅-C₁₀-Heteroaryl enthaltend mindestens ein Heteroatom ausgewählt aus N, O und S;
Y¹, Y², Y³ sind unabhängig voneinander eine Bindung, unsubstituiertes Methylen, Ethylen, Trimethylen, Tetramethylen, Pentamethylen oder Hexamethylen.

3. Verfahren nach Anspruch 1 oder 2, wobei der Komplexkatalysator mindestens einen Phosphordonorliganden der allgemeinen Formel (V) enthält: 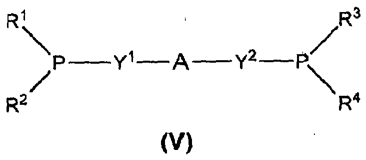 wobei
R¹, R², R³, R⁴ sind unabhängig voneinander unsubstituiertes oder zumindest monosubstituiertes C₁-C₁₀-Alkyl, C₁-C₄-Alkyldiphenylphosphin (-C₁-C₄-Alkyl-P(Phenyl)₂), C₃-C₁₀-Cycloalkyl, C₃-C₁₀-Heterocyclyl enthaltend mindestens ein Heteroatom ausgewählt aus N, O und S, C₅-C₁₄-Aryl oder C₅-C₁₀-Heteroaryl enthaltend mindestens ein Heteroatom ausgewählt aus N, O und S,
wobei die Substituenten ausgewählt sind aus der Gruppe bestehend aus: F, Cl, Br, OH, CN, NH₂ und C₁-C₁₀-Alkyl;
A ist
i) eine Brückengruppe ausgewählt aus der Gruppe unsubstituiertes oder zumindest monosubstituiertes N, P, C₁-C₆-Alkan, C₃-C₁₀-Cycloalkan, C₃-C₁₀-Heterocycloalkan enthaltend mindestens ein Heteroatom ausgewählt aus N, O und S, C₅-C₆-Heteroaromat enthaltend mindestens ein Heteroatom ausgewählt aus N, O und S und C₅-C₁₄-Aromat,
wobei die Substituenten ausgewählt sind aus der Gruppe bestehend aus: C₁-C₄-Alkyl, Phenyl, F, Cl, Br, OH, OR⁷, NH₂, NHR⁷ oder N(R⁷)₂,
wobei R⁷ ausgewählt ist aus C₁-C₁₀-Alkyl und C₅-C₁₀-Aryl;
oder
ii) eine Brückengruppe der Formel II oder III: 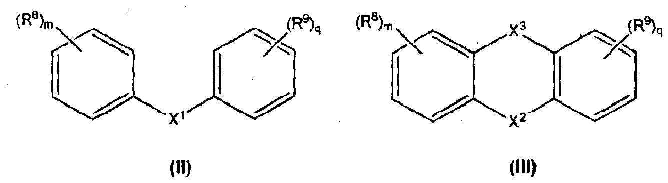
m, q sind unabhängig voneinander 0, 1, 2, 3 oder 4;
R⁸, R⁹ sind unabhängig voneinander ausgewählt aus der Gruppe C₁-C₁₀-Alkyl, F, Cl, Br, OH, OR⁷, NH₂, NHR⁷ und N(R⁷)₂,
wobei R⁷ ausgewählt ist aus C₁-C₁₀-Alkyl und C₅-C₁₀-Aryl;
X¹, X² sind unabhängig voneinander NH, O oder S,
X³ ist eine Bindung, NH, NR¹⁰, O, S oder CR¹¹R¹²;
R¹⁰ ist unsubstituiertes oder zumindest monosubstituiertes C₁-C₁₀-Alkyl, C₃-C₁₀-Cycloalkyl, C₃-C₁₀-Heterocyclyl enthaltend mindestens ein Heteroatom ausgewählt aus N, O und S, C₅-C₁₄-Aryl oder C₅-C₁₀-Heteroaryl enthaltend mindestens ein Heteroatom ausgewählt aus N, O und S,
wobei die Substituenten ausgewählt sind aus der Gruppe bestehend aus: F, Cl, Br, OH, CN, NH₂ und C₁-C₁₀-Alkyl;
R¹¹, R¹² sind unabhängig voneinander unsubstituiertes oder zumindest monosubstituiertes C₁-C₁₀-Alkyl, C₁-C₁₀-Alkoxy, C₃-C₁₀-Cycloalkyl, C₃-C₁₀-Cycloalkoxy,C₃-C₁₀-Heterocyclyl enthaltend mindestens ein Heteroatom ausgewählt aus N, O und S, C₅-C₁₄-Aryl, C₅-C₁₄-Aryloxy oder C₅-C₁₀-Heteroaryl enthaltend mindestens ein Heteroatom ausgewählt aus N, O und S,
wobei die Substituenten ausgewählt sind aus der Gruppe bestehend aus: F, Cl, Br, OH, CN, NH₂ und C₁-C₁₀-Alkyl;
Y¹, Y² sind unabhängig voneinander eine Bindung, unsubstituiertes oder zumindest monosubstituiertes Methylen, Ethylen, Trimethylen, Tetramethylen, Pentamethylen oder Hexamethylen,
wobei die Substituenten ausgewählt sind aus der Gruppe bestehend aus: F, Cl, Br, OH, OR⁷, CN, NH₂, NHR⁷, N(R⁷)₂ und C₁-C₁₀-Alkyl,
wobei R⁷ ausgewählt ist aus C₁-C₁₀-Alkyl und C₅-C₁₀-Aryl.

4. Verfahren nach Anspruch 1 oder 2, wobei der Komplexkatalysator mindestens einen Phosphordonorliganden der allgemeinen Formel (VI) enthält: 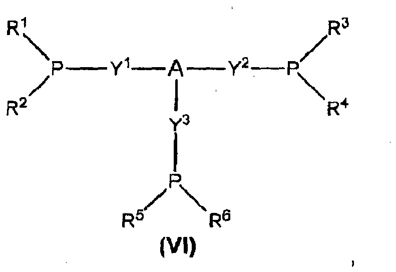 wobei
R¹, R², R³, R⁴, R⁵, R⁶ sind unabhängig voneinander unsubstituiertes oder zumindest monosubstituiertes C₁-C₁₀-Alkyl, C₃-C₁₀-Cycloalkyl, C₃-C₁₀-Heterocyclyl enthaltend mindestens ein Heteroatom ausgewählt aus N, O und S, C₅-C₁₄-Aryl oder C₅-C₁₀-Heteroaryl enthaltend mindestens ein Heteroatom ausgewählt aus N, O und S,
wobei die Substituenten ausgewählt sind aus der Gruppe bestehend aus: F, Cl, Br, OH, CN, NH₂ und C₁-C₁₀-Alkyl;
A ist eine Brückengruppe ausgewählt aus der Gruppe unsubstituiertes oder zumindest monosubstituiertes N, P, C₁-C₈-Alkan, C₃-C₁₀-Cycloalkan, C₃-C₁₀-Heterocycloalkan enthaltend mindestens ein Heteroatom ausgewählt aus N, O und S, C₅-C₆-Heteroaromat enthaltend mindestens ein Heteroatom ausgewählt aus N, O und S und C₅-C₁₄-Aromat,
wobei die Substituenten ausgewählt sind aus der Gruppe bestehend aus: C₁-C₄-Alkyl, F, Cl, Br, OH, OR⁷, NH₂, NHR⁷ oder N(R⁷)₂,
wobei R⁷ ausgewählt ist aus C₁-C₁₀-Alkyl und C₅-C₁₀-Aryl;
Y¹, Y², Y³ sind unabhängig voneinander eine Bindung, unsubstituiertes oder zumindest monosubstituiertes Methylen, Ethylen, Trimethylen, Tetramethylen, Pentamethylen oder Hexamethylen,
wobei die Substituenten ausgewählt sind aus der Gruppe bestehend aus: F, Cl, Br, OH, OR⁷, CN, NH₂, NHR⁷, N(R⁷)₂ und C₁-C₁₀-Alkyl,
wobei R⁷ ausgewählt ist aus C₁-C₁₀-Alkyl und C₅-C₁₀-Aryl.

5. Verfahren nach einem der Ansprüche 1 bis 3, wobei der Komplexkatalysator mindestens einen Phosphordonorliganden der allgemeinen Formel (IX) oder (X) enthält, 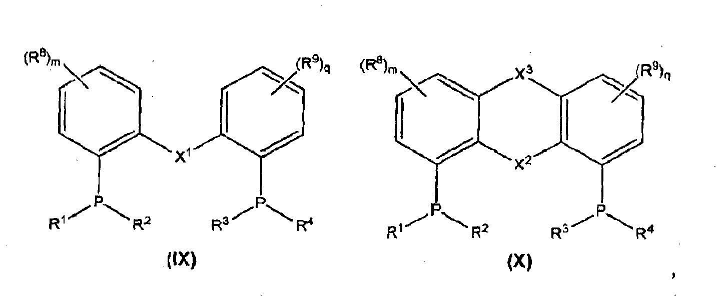 wobei m, q, R¹, R², R³, R⁴, R⁸, R⁹, X¹, X² und X³ die in Anspruch 1 genannten Bedeutungen aufweisen.

6. Verfahren nach einem der Ansprüche 1 oder 2, wobei der Komplexkatalysator mindestens ein Element ausgewählt aus der Gruppe Ruthenium und Iridium sowie mindestens einen Phosphordonorliganden ausgewählt aus der Gruppe 1,2-Bis(diphenylphosphino)ethan (dppe), 1.2-Bis(dicylohexylphosphino)ethan, 1,2-Bis(diphenylphosphino)propan (dppp), 1,2-Bis(diphenytphosphino)butan (dppb), 2,3-Bis(dicyclohexylphosphino)ethan (dcpe), 4,5-Bis(dlphenylphosphino)-9,9-dimethylxanthen (Xantphos), Bis(2-diphenylphosphinaethyl)phenylphosphin und 1,1,1-Tris(Diphenylphosphinomethyl)ethan (Triphos) enthält.

7. Verfahren nach einem der Ansprüche 1 oder 2, wobei der Komplexkatalysator Ruthenium sowie mindestens einen Phosphordonorliganden ausgewählt aus der Gruppe 4,5-Bis(diphenylphosphino)-9,9-dimethylxanthen (Xantphos), Bis(2-diphenylphosphinoethyl)phenylphosphin und 1,1,1-Tris(Diphenylphosphino-methyl)ethan (Triphos) enthält.

8. Verfahren nach Anspruch 7, wobei der Komplexkatalysator Ruthenium und einen Phosphordonorliganden ausgewählt aus der Gruppe Bis(2-diphenylphosphino-ethyl)phenylphosphin und Triphos enthält.

9. Verfahren nach einem der Ansprüche 1 oder 2, wobei der Komplexkatalysator Iridium sowie mindestens einen Phosphordonorliganden ausgewählt aus der Gruppe 4,5-Bis(diphenylphosphino)-9,9-dimethylxanthen (Xantphos), Bis(2-diphenylphosphinoethyl)phenylphosphin und 1,1,1-Tris(Diphenylphosphinomethyl)ethan (Triphos) enthält.

10. Verfahren nach einem der Ansprüche 1 bis 9, wobei das Edukt mindestens zwei funktiontionelle Gruppen der Formel (-CH₂-OH) enthält.

11. Verfahren nach einem der Ansprüche 1 bis 10, wobei die Alkoholaminierung in Gegenwart eines unpolaren Lösungsmittels durchgeführt wird.

12. Verfahren nach Anspruch 11, wobei das unpolare Lösungsmittel ausgewählt ist aus der Gruppe bestehend aus gesättigtem Hexan, Heptan, Oktan, Cyclohexan, Benzol, Toluol, Xylol, Mesltylen, THF, Diethylether, 1,4-Dioxan, MTBE, Diglyme und 1,2-Dimethoxyethan.

13. Verfahren nach einem der Ansprüche 1 bis 12, wobei die Alkoholaminierung bei 20 bis 250 °C und bei Drücken von 0,1 bis 20 MPas absolut durchgeführt wird:

14. Verfahren nach einem der Ansprüche 1 bis 13, wobei die Alkoholaminierung unter Zusatz von Basen erfolgt

15. Verwendung eines Komplexkatalysators, der mindestens ein Element ausgewählt aus den Gruppen 8 und 9 des Periodensystems sowie mindestens einen Phosphordonorliganden der allgemeinen Formel (I) enthält, 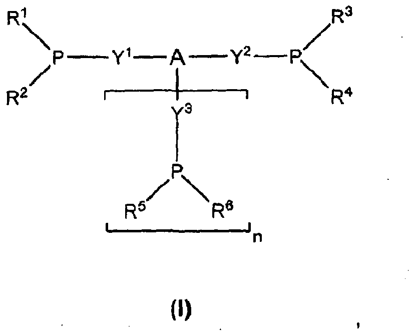 wobei A, R¹, R², R³, R⁴, R⁵, R⁶, Y¹, Y² und Y³ die in Anspruch 1 genannten Bedeutungen aufweisen, zur homogen-katalysierten Herstellung von primären Aminen, die mindestens eine funktionelle Gruppe der Formel (-CH₂-NH₂) aufweisen, durch Alkoholaminierung von Edukten, die mindestens eine funktionelle Gruppe der Formel (-CH₂-OH) aufweisen, mit Ammoniak.

## Claims

1. A process for the preparation of primary amines which have at least one functional group of the formula (-CH₂-NH₂) by alcohol amination of starting materials which have at least one functional group of the formula (-CH₂-OH), with ammonia, with the elimination of water, where the alcohol amination is carried out under homogeneous catalysis in the presence of at least one complex catalyst which comprises at least one element selected from groups 8 and 9 of the Periodic Table of the Elements, and also at least one phosphorus donor ligand of the general formula (I), 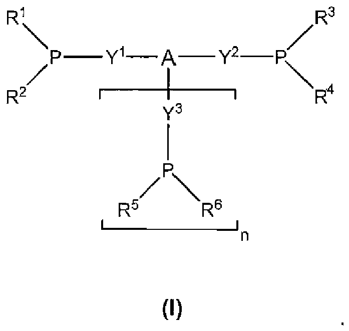 where
n is 0 or 1;
R¹, R², R³, R⁴, R⁵, R⁶ are, independently of one another, unsubstituted or at least monosubstituted C₁-C₁₀-alkyl, C₁-C₄-alkyldiphenylphosphine (-C₁-C₄-alkyl-P(phenyl)₂), C₃-C₁₀-cycloalkyl, C₃-C₁₀-heterocyclyl comprising at least one heteroatom selected from N, O and S, C₅-C₁₄-aryl or C₅-C₁₀-heteroaryl comprising at least one heteroatom selected from N, O and S,
where the substituents are selected from the group consisting of: F, Cl, Br, OH, CN, NH₂ and C₁-C₁₀-alkyl;
A is
i) a bridging group selected from the group unsubstituted or at least monosubstituted N, O, P, C₁-C₆-alkane, C₃-C₁₀-cycloalkane, C₃-C₁₀-heterocycloalkane comprising at least one heteroatom selected from N, O and S, C₅-C₆-heteroaromatic comprising at least one heteroatom selected from N, O and S and C₅-C₁₄-aromatic,
where the substituents are selected from the group consisting of: C₁-C₄-alkyl, phenyl, F, Cl, Br, OH, OR⁷, NH₂, NHR⁷ or N(R⁷)₂,
where R⁷ is selected from C₁-C₁₀-alkyl and C₅-C₁₀-aryl;
or
ii) a bridging group of the formula (II) or (III): 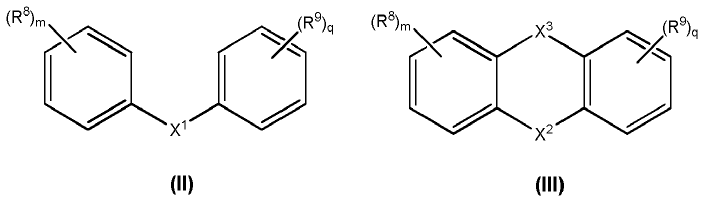
m, q are, independently of one another, 0, 1, 2, 3 or 4;
R⁸, R⁹ are, independently of one another, selected from the group C₁-C₁₀-alkyl, F, Cl, Br, OH, OR⁷, NH₂, NHR⁷ and N(R⁷)₂,
where R⁷ is selected from C₁-C₁₀-alkyl and C₅-C₁₀-aryl;
X¹, X² are, independently of one another, NH, O or S;
X³ is a bond, NH, NR¹⁰, O, S or CR¹¹R¹²;
R¹⁰ is unsubstituted or at least monosubstituted C₁-C₁₀-alkyl, C₃-C₁₀-cycloalkyl, C₃-C₁₀-heterocyclyl comprising at least one heteroatom selected from N, O and S, C₅-C₁₄-aryl or C₅-C₁₀-heteroaryl comprising at least one heteroatom selected from N, O and S,
where the substituents are selected from the group consisting of: F, Cl, Br, OH, CN, NH₂ and C₁-C₁₀-alkyl;
R¹¹, R¹² are, independently of one another, unsubstituted or at least monosubstituted C₁-C₁₀-alkyl, C₁-C₁₀-alkoxy, C₃-C₁₀-cycloalkyl, C₃-C₁₀-cycloalkoxy, C₃-C₁₀-heterocyclyl comprising at least one heteroatom selected from N, O and S, C₅-C₁₄-aryl, C₅-C₁₄-aryloxy or C₅-C₁₀-heteroaryl comprising at least one heteroatom selected from N, O and S,
where the substituents are selected from the group consisting of: F, Cl, Br, OH, CN, NH₂ and C₁-C₁₀-alkyl;
Y¹, Y², Y³ are, independently of one another, a bond, unsubstituted or
at least monosubstituted methylene, ethylene, trimethylene, tetramethylene, pentamethylene or hexamethylene, where the substituents are selected from the group consisting of: F, Cl, Br, OH, OR⁷, CN, NH₂, NHR⁷, N(R⁷)₂ and C₁-C₁₀-alkyl,
where R⁷ is selected from C₁-C₁₀-alkyl and C₅-C₁₀-aryl.

2. The process according to claim 1, where
n is 0 or 1;
R¹, R², R³, R⁴, R⁵, R⁶ are, independently of one another, unsubstituted C₁-C₁₀-alkyl, C₁-C₄-alkyldiphenylphosphine, C₃-C₁₀-cycloalkyl, C₃-C₁₀-heterocyclyl comprising at least one heteroatom selected from N, O and S, C₅-C₁₄-aryl or C₅-C₁₀-heteroaryl comprising at least one heteroatom selected from N, O and S;
A is
i) a bridging group selected from the group unsubstituted N, O, P, C₁-C₆-alkane, C₃-C₁₀-cycloalkane, C₃-C₁₀-heterocycloalkane comprising at least one heteroatom selected from N, O and S, C₅-C₆-heteroaromatic comprising at least one heteroatom selected from N, O and S and C₅-C₁₄-aromatic;
or
ii) a bridging group of the formula II or III: 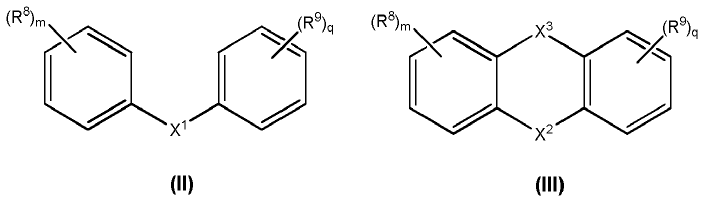
m, q are, independently of one another, 0, 1, 2, 3 or 4;
R⁸, R⁹ are, independently of one another, selected from the group C₁-C₁₀-alkyl, F, Cl, Br, OH, OR⁷, NH₂, NHR⁷ and N(R⁷)₂,
where R⁷ is selected from C₁-C₁₀-alkyl and C₅-C₁₀-aryl;
X¹, X² are, independently of one another, NH, O or S;
X³ is a bond, NH, NR¹⁰, O, S or CR¹¹R¹²;
R¹⁰ is unsubstituted C₁-C₁₀-alkyl, C₃-C₁₀-cycloalkyl, C₃-C₁₀-heterocyclyl comprising at least one heteroatom selected from N, O and S, C₅-C₁₄-aryl or C₅-C₁₀-heteroaryl comprising at least one heteroatom selected from N, O and S;
R¹¹, R¹² are, independently of one another, unsubstituted C₁-C₁₀-alkyl, C₁-C₁₀-alkoxy, C₃-C₁₀-cycloalkyl, C₃-C₁₀-cycloalkoxy, C₃-C₁₀-heterocyclyl comprising at least one heteroatom selected from N, O and S, C₅-C₁₄-aryl, C₅-C₁₄-aryloxy or C₅-C₁₀-heteroaryl comprising at least one heteroatom selected from N, O and S;
Y¹, Y², Y³ are, independently of one another, a bond, unsubstituted methylene, ethylene, trimethylene, tetramethylene, pentamethylene or hexamethylene.

3. The process according to claim 1 or 2, where the complex catalyst comprises at least one phosphorus donor ligand of the general formula (V): 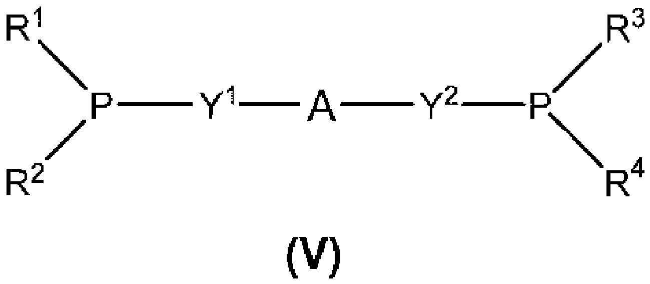 where
R¹, R², R³, R⁴ are, independently of one another, unsubstituted or at least monosubstituted C₁-C₁₀-alkyl, C₁-C₄-alkyldiphenylphosphine (-C₁-C₄-alkyl-P(phenyl)₂), C₃-C₁₀-cycloalkyl, C₃-C₁₀-heterocyclyl comprising at least one heteroatom selected from N, O and S, C₅-C₁₄-aryl or C₅-C₁₀-heteroaryl comprising at least one heteroatom selected from N, O and S,
where the substituents are selected from the group consisting of: F, Cl, Br, OH, CN, NH₂ and C₁-C₁₀-alkyl;
A is
i) a bridging group selected from the group unsubstituted or at least monosubstituted N, P, C₁-C₆-alkane, C₃-C₁₀-cycloalkane, C₃-C₁₀-heterocycloalkane comprising at least one heteroatom selected from N, O and S, C₅-C₆-heteroaromatic comprising at least one heteroatom selected from N, O and S and C₅-C₁₄-aromatic,
where the substituents are selected from the group consisting of: C₁-C₄-alkyl, phenyl, F, Cl, Br, OH, OR⁷, NH₂, NHR⁷ or N(R⁷)₂,
where R⁷ is selected from C₁-C₁₀-alkyl and C₅-C₁₀-aryl;
or
ii) a bridging group of the formula II or III: 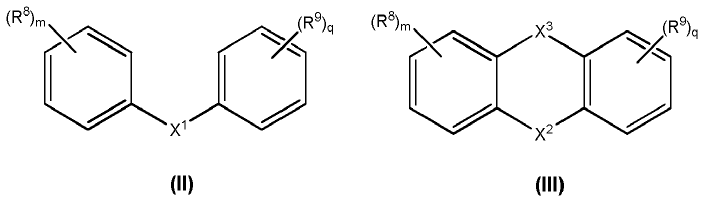
m, q are, independently of one another, 0, 1, 2, 3 or 4;
R⁸, R⁹ are, independently of one another, selected from the group C₁-C₁₀-alkyl, F, Cl, Br, OH, OR⁷, NH₂, NHR⁷ and N(R⁷)₂,
where R⁷ is selected from C₁-C₁₀-alkyl and C₅-C₁₀-aryl;
X¹, X² are, independently of one another, NH, O or S;
X³ is a bond, NH, NR¹⁰, O, S or CR¹¹R¹²;
R¹⁰ is unsubstituted or at least monosubstituted C₁-C₁₀-alkyl, C₃-C₁₀-cycloalkyl, C₃-C₁₀-heterocyclyl comprising at least one heteroatom selected from N, O and S, C₅-C₁₄-aryl or C₅-C₁₀-heteroaryl comprising at least one heteroatom selected from N, O and S,
where the substituents are selected from the group consisting of: F, Cl, Br, OH, CN, NH₂ and C₁-C₁₀-alkyl;
R¹¹, R¹² are, independently of one another, unsubstituted or at least monosubstituted C₁-C₁₀-alkyl, C₁-C₁₀-alkoxy, C₃-C₁₀-cycloalkyl, C₃-C₁₀-cycloalkoxy, C₃-C₁₀-heterocyclyl comprising at least one heteroatom selected from N, O and S, C₅-C₁₄-aryl, C₅-C₁₄-aryloxy or C₅-C₁₀-heteroaryl comprising at least one heteroatom selected from N, O and S,
where the substituents are selected from the group consisting of: F, Cl, Br, OH, CN, NH₂ and C₁-C₁₀-alkyl;
Y¹, Y² are, independently of one another, a bond, unsubstituted or at least monosubstituted methylene, ethylene, trimethylene, tetramethylene, pentamethylene or hexamethylene,
where the substituents are selected from the group consisting of: F, Cl, Br, OH, OR⁷, CN, NH₂, NHR⁷, N(R⁷)₂ and C₁-C₁₀-alkyl,
where R⁷ is selected from C₁-C₁₀-alkyl and C₅-C₁₀-aryl.

4. The process according to claim 1 or 2, where the complex catalyst comprises at least one phosphorus donor ligand of the general formula (VI): 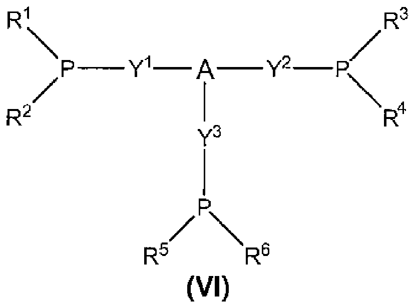 where
R¹, R², R³, R⁴, R⁵, R⁶ are, independently of one another, unsubstituted or at least monosubstituted C₁-C₁₀-alkyl, C₃-C₁₀-cycloalkyl, C₃-C₁₀-heterocyclyl comprising at least one heteroatom selected from N, O and S, C₅-C₁₄-aryl or C₅-C₁₀-heteroaryl comprising at least one heteroatom selected from N, O and S,
where the substituents are selected from the group consisting of: F, Cl, Br, OH, CN, NH₂ and C₁-C₁₀-alkyl;
A is a bridging group selected from the group unsubstituted or at least monosubstituted N, P, C₁-C₆-alkane, C₃-C₁₀-cycloalkane, C₃-C₁₀-heterocycloalkane comprising at least one heteroatom selected from N, O and S, C₅-C₆-heteroaromatic comprising at least one heteroatom selected from N, O and S and C₅-C₁₄-aromatic,
where the substituents are selected from the group consisting of: C₁-C₄-alkyl, F, Cl, Br, OH, OR⁷, NH₂, NHR⁷ or N(R⁷)₂,
where R⁷ is selected from C₁-C₁₀-alkyl and C₅-C₁₀-aryl;
Y¹, Y², Y³ are, independently of one another, a bond, unsubstituted or at least monosubstituted methylene, ethylene, trimethylene, tetramethylene, pentamethylene or hexamethylene,
where the substituents are selected from the group consisting of: F, Cl, Br, OH, OR⁷, CN, NH₂, NHR⁷, N(R⁷)₂ and C₁-C₁₀-alkyl,
where R⁷ is selected from C₁-C₁₀-alkyl and C₅-C₁₀-aryl.

5. The process according to any one of claims 1 to 3, where the complex catalyst comprises at least one phosphorus donor ligand of the general formula (IX) or (X), 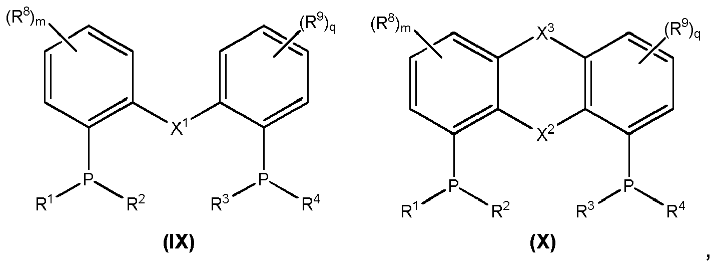 where m, q, R¹, R², R³, R⁴, R⁸, R⁹, X¹, X² and X³ have the meanings given in claim 1.

6. The process according to either of claims 1 or 2, where the complex catalyst comprises at least one element selected from the group ruthenium and iridium, and also at least one phosphorus donor ligand selected from the group 1,2-bis(diphenylphosphino)ethane (dppe), 1,2-bis(dicyclohexylphosphino)ethane, 1,2-bis(diphenylphosphino)propane (dppp), 1,2-bis(diphenylphosphino)butane (dppb), 2,3-bis(dicyclohexylphosphino)ethane (dcpe), 4,5-bis(diphenylphosphino)-9,9-dimethylxanthene (xantphos), bis(2-diphenylphosphinoethyl)phenylphosphine and 1,1,1-tris(diphenylphosphinomethyl)ethane (triphos).

7. The process according to either of claims 1 or 2, where the complex catalyst comprises ruthenium and also at least one phosphorus donor ligand selected from the group 4,5-bis(diphenylphosphino)-9,9-dimethylxanthene (xantphos), bis(2-diphenylphosphinoethyl)phenylphosphine and 1,1,1-tris(diphenylphosphinomethyl)ethane (triphos).

8. The process according to claim 7, where the complex catalyst comprises ruthenium and a phosphorus donor ligand selected from the group bis(2-diphenylphosphinoethyl)phenylphosphine and triphos.

9. The process according to either of claims 1 or 2, where the complex catalyst comprises iridium and also at least one phosphorus donor ligand selected from the group 4,5-bis(diphenylphosphino)-9,9-dimethylxanthene (xantphos), bis(2-diphenylphosphinoethyl)phenylphosphine and 1,1,1-tris(diphenylphosphinomethyl)ethane (triphos).

10. The process according to any one of claims 1 to 9, where the starting material comprises at least two functional groups of the formula (-CH₂-OH).

11. The process according to any one of claims 1 to 10, where the alcohol amination is carried out in the presence of a nonpolar solvent.

12. The process according to claim 11, where the nonpolar solvent is selected from the group consisting of saturated hexane, heptane, octane, cyclohexane, benzene, toluene, xylene, mesitylene, THF, diethyl ether, 1,4-dioxane, MTBE, diglyme and 1,2-dimethoxyethane.

13. The process according to any one of claims 1 to 12, where the alcohol amination is carried out at 20 to 250°C and at pressures of from 0.1 to 20 MPas absolute.

14. The process according to any one of claims 1 to 13, where the alcohol amination takes place with the addition of bases.

15. The use of a complex catalyst which comprises at least one element selected from groups 8 and 9 of the Periodic Table of the Elements and also at least one phosphorus donor ligand of the general formula (I), 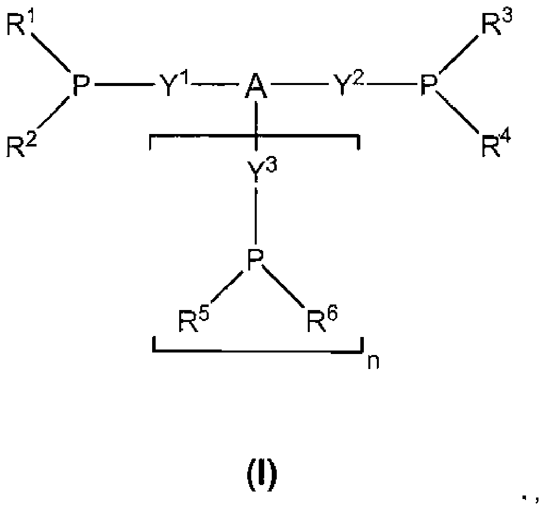 where A, R¹, R², R³, R⁴, R⁵, R⁶, Y¹, Y² and Y³ have the meanings given in claim 1, for the homogeneously catalyzed preparation of primary amines which have at least one functional group of the formula (-CH₂-NH₂), by alcohol amination of starting materials which have at least one functional group of the formula (-CH₂-OH) with ammonia.

## Revendications

1. Procédé de fabrication d'amines primaires comprenant au moins un groupe fonctionnel de formule (-CH₂-NH₂) par amination d'alcool de réactifs comprenant au moins un groupe fonctionnel de formule (-CH₂-OH) avec de l'ammoniac avec clivage d'eau, dans lequel l'amination d'alcool est réalisée sous catalyse homogène en présence d'au moins un catalyseur complexe contenant au moins un élément choisi dans les groupes 8 et 9 du tableau périodique, ainsi qu'au moins un ligand donneur de phosphore de formule générale (I) 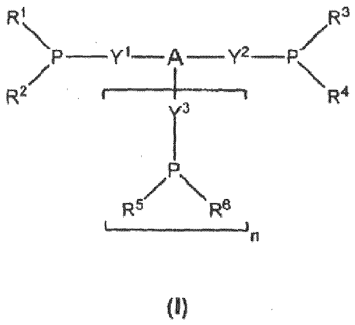 dans laquelle
n représente 0 ou 1,
R¹, R², R³, R⁴, R⁵, R⁶ représentent indépendamment les uns des autres alkyle en C₁-C₁₀, alkyle en C₁-C₄-diphénylphosphine (alkyle en C₁-C₄-P (phényl)₂), cycloalkyle en C₃-C₁₀, hétérocyclyle en C₃-C₁₀ contenant au moins un hétéroatome choisi parmi N, 0 et S, aryle en C₅-C₁₄ ou hétéroaryle en C₅-C₁₀ contenant au moins un hétéroatome choisi parmi N, 0 et S, non substitué ou au moins monosubstitué,
les substituants étant choisis dans le groupe constitué par : F, Cl, Br, OH, CN, NH₂ et alkyle en C₁-C₁₀ ;
A représente
i) un groupe de pontage choisi dans le groupe constitué par N, 0, P, alcane en C₁-C₆, cycloalcane en C₃-C₁₀, hétérocycloalcane en C₃-C₁₀ contenant au moins un hétéroatome choisi parmi N, 0 et S, hétéroaromatique en C₅-C₆ contenant au moins un hétéroatome choisi parmi N, O et S, et aromatique en C₅-C₁₄, non substitué ou au moins monosubstitué,
les substituants étant choisis dans le groupe constitué par : alkyle en C₁-C₄, phényle, F, Cl, Br, OH, OR⁷, NH₂, NHR⁷ ou N(R⁷)₂,
R⁷ étant choisi parmi alkyle en C₁-C₁₀ et aryle en C₅-C₁₀ ;
ou
ii) un groupe de pontage de formule (II) ou (III) : 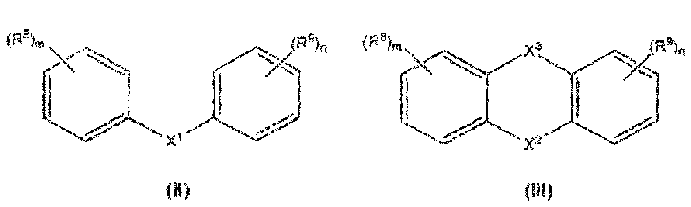
m, q représentent indépendamment l'un de l'autre 0, 1, 2, 3 ou 4,
R⁸, R⁹ sont choisis indépendamment l'un de l'autre dans le groupe constitué par alkyle en C₁-C₁₀, F, Cl, Br, OH, OR⁷, NH₂, NHR⁷ et N(R⁷)₂,
R⁷ étant choisi parmi alkyle en C₁-C₁₀ et aryle en C₅-C₁₀ ;
X¹, X² représentent indépendamment l'un de l'autre NH, O ou S,
X³ représente une liaison, NH, NR¹⁰, 0, S ou CR¹¹R¹², R¹⁰ représente alkyle en C₁-C₁₀, cycloalkyle en C₃-C₁₀, hétérocyclyle en C₃-C₁₀ contenant au moins un hétéroatome choisi parmi N, 0 et S, aryle en C₅-C₁₄ ou hétéroaryle en C₅-C₁₀ contenant au moins un hétéroatome choisi parmi N, 0 et S, non substitué ou au moins monosubstitué,
les substituants étant choisis dans le groupe constitué par : F, Cl, Br, OH, CN, NH₂ et alkyle en C₁-C₁₀ ;
R¹¹, R¹² représentent indépendamment l'un de l'autre alkyle en C₁-C₁₀, alcoxy en C₁-C₁₀, cycloalkyle en C₃-C₁₀, cycloalcoxy en C₃-C₁₀, hétérocyclyle en C₃-C₁₀ contenant au moins un hétéroatome choisi parmi N, 0 et S, aryle en C₅-C₁₄, aryloxy en C₅-C₁₄ ou hétéroaryle en C₅-C₁₀ contenant au moins un hétéroatome choisi parmi N, 0 et S, non substitué ou au moins monosubstitué,
les substituants étant choisis dans le groupe constitué par : F, Cl, Br, OH, CN, NH₂ et alkyle en C₁-C₁₀ ;
Y¹, Y², Y³ représentent indépendamment les uns des autres une liaison, méthylène, éthylène, triméthylène, tétraméthylène, pentaméthylène ou hexaméthylène non substitué ou au moins monosubstitué,
les substituants étant choisis dans le groupe constitué par : F, Cl, Br, OH, OR⁷, CN, NH₂, NHR⁷, N(R⁷)₂ et alkyle en C₁-C₁₀,
R⁷ étant choisi parmi alkyle en C₁-C₁₀ et aryle en C₅-C₁₀.

2. Procédé selon la revendication 1, dans lequel
n représente 0 ou 1 ;
R¹, R², R³, R⁴, R⁵, R⁶ représentent indépendamment les uns des autres alkyle en C₁-C₁₀, alkyle en C₁-C₄-diphénylphosphine, cycloalkyle en C₃-C₁₀, hétérocyclyle en C₃-C₁₀ contenant au moins un hétéroatome choisi parmi N, 0 et S, aryle en C₅-C₁₄ ou hétéroaryle en C₅-C₁₀ contenant au moins un hétéroatome choisi parmi N, 0 et S, non substitué,
A représente
i) un groupe de pontage choisi dans le groupe constitué par N, 0, P, alcane en C₁-C₆, cycloalcane en C₃-C₁₀, hétérocycloalcane en C₃-C₁₀ contenant au moins un hétéroatome choisi parmi N, 0 et S, hétéroaromatique en C₅-C₆ contenant au moins un hétéroatome choisi parmi N, O et S, et aromatique en C₅-C₁₄, non substitué,
ou
ii) un groupe de pontage de formule II ou III : 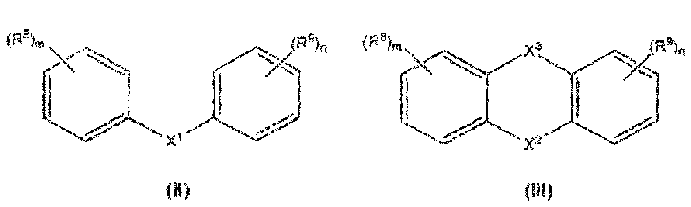 m, q représentent indépendamment l'un de l'autre 0, 1, 2, 3 ou 4,
R⁸, R⁹ sont choisis indépendamment l'un de l'autre dans le groupe constitué par alkyle en C₁-C₁₀, F, Cl, Br, OH, OR⁷, NH₂, NHR⁷ et N(R⁷)₂,
R⁷ étant choisi parmi alkyle en C₁-C₁₀ et aryle en C₅-C₁₀ ;
X¹, X² représentent indépendamment l'un de l'autre NH, O ou S,
X³ représente une liaison, NH, NR¹⁰, 0, S ou CR¹¹R¹²,
R¹⁰ représente alkyle en C₁-C₁₀, cycloalkyle en C₃-C₁₀, hétérocyclyle en C₃-C₁₀ contenant au moins un hétéroatome choisi parmi N, 0 et S, aryle en C₅-C₁₄ ou hétéroaryle en C₅-C₁₀ contenant au moins un hétéroatome choisi parmi N, 0 et S, non substitué,
R¹¹, R¹² représentent indépendamment l'un de l'autre alkyle en C₁-C₁₀, alcoxy en C₁-C₁₀, cycloalkyle en C₃-C₁₀, cycloalcoxy en C₃-C₁₀, hétérocyclyle en C₃-C₁₀ contenant au moins un hétéroatome choisi parmi N, 0 et S, aryle en C₅-C₁₄, aryloxy en C₅-C₁₄ ou hétéroaryle en C₅-C₁₀ contenant au moins un hétéroatome choisi parmi N, O et S, non substitué,
Y¹, Y², Y³ représentent indépendamment les uns des autres une liaison, méthylène, éthylène, triméthylène, tétraméthylène, pentaméthylène ou hexaméthylène non substitué.

3. Procédé selon la revendication 1 ou 2, dans lequel le catalyseur complexe contient au moins un ligand donneur de phosphore de formule générale (V) : 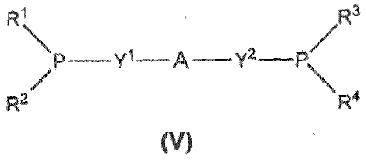 dans laquelle
R¹, R², R³, R⁴ représentent indépendamment les uns des autres alkyle en C₁-C₁₀, alkyle en C₁-C₄-diphénylphosphine (alkyle en C₁-C₄-P (phényl)₂), cycloalkyle en C₃-C₁₀, hétérocyclyle en C₃-C₁₀ contenant au moins un hétéroatome choisi parmi N, 0 et S, aryle en C₅-C₁₄ ou hétéroaryle en C₅-C₁₀ contenant au moins un hétéroatome choisi parmi N, 0 et S, non substitué ou au moins monosubstitué,
les substituants étant choisis dans le groupe constitué par : F, Cl, Br, OH, CN, NH₂ et alkyle en C₁-C₁₀ ;
A représente
i) un groupe de pontage choisi dans le groupe constitué par N, P, alcane en C₁-C₆, cycloalcane en C₃-C₁₀, hétérocycloalcane en C₃-C₁₀ contenant au moins un hétéroatome choisi parmi N, 0 et S, hétéroaromatique en C₅-C₆ contenant au moins un hétéroatome choisi parmi N, O et S, et aromatique en C₅-C₁₄, non substitué ou au moins monosubstitué,
les substituants étant choisis dans le groupe constitué par : alkyle en C₁-C₄, phényle, F, Cl, Br, OH, OR⁷, NH₂, NHR⁷ ou N(R⁷)₂,
R⁷ étant choisi parmi alkyle en C₁-C₁₀ et aryle en C₅-C₁₀ ;
ou
ii) un groupe de pontage de formule II ou III : 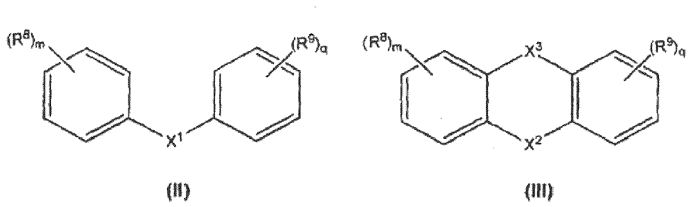 m, q représentent indépendamment l'un de l'autre 0, 1, 2, 3 ou 4,
R⁸, R⁹ sont choisis indépendamment l'un de l'autre dans le groupe constitué par alkyle en C₁-C₁₀, F, Cl, Br, OH, OR⁷, NH₂, NHR⁷ et N(R⁷)₂,
R⁷ étant choisi parmi alkyle en C₁-C₁₀ et aryle en C₅-C₁₀ ;
X¹, X² représentent indépendamment l'un de l'autre NH, O ou S,
X³ représente une liaison, NH, NR¹⁰, 0, S ou CR¹¹R¹²,
R¹⁰ représente alkyle en C₁-C₁₀, cycloalkyle en C₃-C₁₀, hétérocyclyle en C₃-C₁₀ contenant au moins un hétéroatome choisi parmi N, 0 et S, aryle en C₅-C₁₄ ou hétéroaryle en C₅-C₁₀ contenant au moins un hétéroatome choisi parmi N, 0 et S, non substitué ou au moins monosubstitué,
les substituants étant choisis dans le groupe constitué par : F, Cl, Br, OH, CN, NH₂ et alkyle en C₁-C₁₀ ;
R¹¹, R¹² représentent indépendamment l'un de l'autre alkyle en C₁-C₁₀, alcoxy en C₁-C₁₀, cycloalkyle en C₃-C₁₀, cycloalcoxy en C₃-C₁₀, hétérocyclyle en C₃-C₁₀ contenant au moins un hétéroatome choisi parmi N, 0 et S, aryle en C₅-C₁₄, aryloxy en C₅-C₁₄ ou hétéroaryle en C₅-C₁₀ contenant au moins un hétéroatome choisi parmi N, 0 et S, non substitué ou au moins monosubstitué,
les substituants étant choisis dans le groupe constitué par : F, Cl, Br, OH, CN, NH₂ et alkyle en C₁-C₁₀ ;
Y¹, Y² représentent indépendamment les uns des autres une liaison, méthylène, éthylène, triméthylène, tétraméthylène, pentaméthylène ou hexaméthylène non substitué ou au moins monosubstitué,
les substituants étant choisis dans le groupe constitué par : F, Cl, Br, OH, OR⁷, CN, NH₂, NHR⁷, N(R⁷)₂ et alkyle en C₁-C₁₀,
R⁷ étant choisi parmi alkyle en C₁-C₁₀ et aryle en C₅-C₁₀.

4. Procédé selon la revendication 1 ou 2, dans lequel le catalyseur complexe contient au moins un ligand donneur de phosphore de formule générale (VI) : 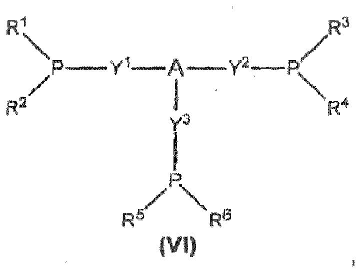 dans laquelle
R¹, R², R³, R⁴, R⁵, R⁶ représentent indépendamment les uns des autres alkyle en C₁-C₁₀, cycloalkyle en C₃-C₁₀, hétérocyclyle en C₃-C₁₀ contenant au moins un hétéroatome choisi parmi N, 0 et S, aryle en C₅-C₁₄ ou hétéroaryle en C₅-C₁₀ contenant au moins un hétéroatome choisi parmi N, 0 et S, non substitué ou au moins monosubstitué,
les substituants étant choisis dans le groupe constitué par : F, Cl, Br, OH, CN, NH₂ et alkyle en C₁-C₁₀ ;
A représente un groupe de pontage choisi dans le groupe constitué par N, P, alcane en C₁-C₆, cycloalcane en C₃-C₁₀, hétérocycloalcane en C₃-C₁₀ contenant au moins un hétéroatome choisi parmi N, 0 et S, hétéroaromatique en C₅-C₆ contenant au moins un hétéroatome choisi parmi N, 0 et S, et aromatique en C₅-C₁₄, non substitué ou au moins monosubstitué,
les substituants étant choisis dans le groupe constitué par : alkyle en C₁-C₄, F, Cl, Br, OH, OR⁷, NH₂, NHR⁷ ou N(R⁷)₂,
R⁷ étant choisi parmi alkyle en C₁-C₁₀ et aryle en C₅-C₁₀ ;
Y¹, Y², Y³ représentent indépendamment les uns des autres une liaison, méthylène, éthylène, triméthylène, tétraméthylène, pentaméthylène ou hexaméthylène non substitué ou au moins monosubstitué,
les substituants étant choisis dans le groupe constitué par : F, Cl, Br, OH, OR⁷, CN, NH₂, NHR⁷, N(R⁷)₂ et alkyle en C₁-C₁₀,
R⁷ étant choisi parmi alkyle en C₁-C₁₀ et aryle en C₅-C₁₀.

5. Procédé selon l'une quelconque des revendications 1 à 3, dans lequel le catalyseur complexe contient au moins un ligand donneur de phosphore de formule générale (IX) ou (X) 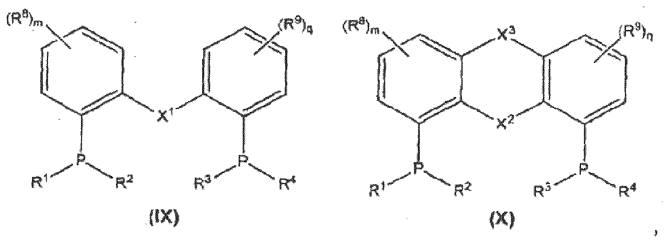 dans laquelle m, q, R¹, R², R³, R⁴, R⁸, R⁹, X¹, X² et X³ ont les significations indiquées dans la revendication 1.

6. Procédé selon l'une quelconque des revendications 1 ou 2, dans lequel le catalyseur complexe contient au moins un élément choisi dans le groupe constitué par le ruthénium et l'iridium, ainsi qu'au moins un ligand donneur de phosphore choisi dans le groupe constitué par le 1,2-bis(diphénylphosphino)éthane (dppe), le 1,2-bis(dicylohexylphosphino)éthane, le 1,2-bis(diphénylphosphino)propane (dppp), le 1,2-bis(diphénylphosphino)butane (dppb), le 2,3-bis(dicyclohexylphosphino)éthane (dcpe), le 4,5-bis(diphénylphosphino)-9,9-diméthylxanthène (Xantphos), la bis(2-diphénylphosphinoéthyl)phénylphosphine et le 1,1,1-tris(diphénylphosphinométhyl)éthane (Triphos).

7. Procédé selon l'une quelconque des revendications 1 ou 2, dans lequel le catalyseur complexe contient du ruthénium et au moins un ligand donneur de phosphore choisi dans le groupe constitué par le 4,5-bis(diphénylphosphino)-9,9-diméthylxanthène (Xantphos), la bis(2-diphénylphosphinoéthyl)phényl-phosphine et le 1,1,1-tris(diphénylphosphinométhyl)-éthane (Triphos).

8. Procédé selon la revendication 7, dans lequel le catalyseur complexe contient du ruthénium et un ligand donneur de phosphore choisi dans le groupe constitué par la bis(2-diphénylphosphinoéthyl)phénylphosphine et le Triphos.

9. Procédé selon l'une quelconque des revendications 1 ou 2, dans lequel le catalyseur complexe contient de l'iridium et au moins un ligand donneur de phosphore choisi dans le groupe constitué par le 4,5-bis(diphénylphosphino)-9,9-diméthylxanthène (Xantphos), la bis(2-diphénylphosphinoéthyl)phényl-phosphine et le 1,1,1-tris(diphénylphosphinométhyl)-éthane (Triphos).

10. Procédé selon l'une quelconque des revendications 1 à 9, dans lequel le réactif contient au moins deux groupes fonctionnels de formule (-CH₂-OH).

11. Procédé selon l'une quelconque des revendications 1 à 10, dans lequel l'amination d'alcool est réalisée en présence d'un solvant apolaire.

12. Procédé selon la revendication 11, dans lequel le solvant apolaire est choisi dans le groupe constitué par l'hexane saturé, l'heptane, l'octane, le cyclohexane, le benzène, le toluène, le xylène, le mésitylène, le THF, l'éther diéthylique, le 1,4-dioxane, le MTBE, le diglyme et le 1,2-diméthoxyéthane.

13. Procédé selon l'une quelconque des revendications 1 à 12, dans lequel l'amination d'alcool est réalisée à 20 à 250 °C et à des pressions de 0,1 à 20 MPas absolu.

14. Procédé selon l'une quelconque des revendications 1 à 13, dans lequel l'amination d'alcool a lieu avec ajout de bases.

15. Utilisation d'un catalyseur complexe contenant au moins un élément choisi dans les groupes 8 et 9 du tableau périodique et au moins un ligand donneur de phosphore de formule générale (I) 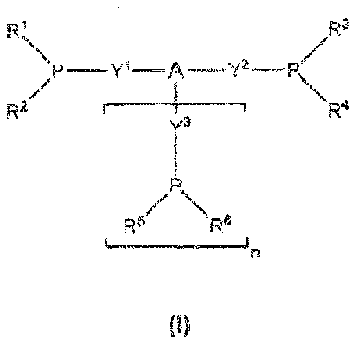 dans laquelle A, R¹, R², R³, R⁴, R⁵, R⁶, Y¹, Y² et Y³ ont les significations indiquées dans la revendication 1, pour la fabrication sous catalyse homogène d'amines primaires comprenant au moins un groupe fonctionnel de formule (-CH₂-NH₂) par amination d'alcool de réactifs comprenant au moins un groupe fonctionnel de formule (-CH₂-OH) avec de l'ammoniac.
